(19) Europäisches Patentamt
European Patent Office
Office européen des brevets

(11) **EP 2 581 394 A1**

(12) **EUROPEAN PATENT APPLICATION**
published in accordance with Art. 153(4) EPC

(43) Date of publication:
**17.04.2013 Bulletin 2013/16**

(21) Application number: **10853203.7**

(22) Date of filing: **14.06.2010**

(51) Int Cl.:
***C08F 20/26*** $^{(2006.01)}$ ***C07C 311/48*** $^{(2006.01)}$
***C07F 7/10*** $^{(2006.01)}$ ***G02C 7/04*** $^{(2006.01)}$

(86) International application number:
**PCT/JP2010/060061**

(87) International publication number:
**WO 2011/158321 (22.12.2011 Gazette 2011/51)**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(71) Applicant: **Menicon Co., Ltd.**
**Nagoya-shi**
**Aichi 460-0006 (JP)**

(72) Inventors:
- **SATAKE Kohsuke**
  **Kasugai-shi, Aichi 487-0032 (JP)**
- **OGAWA Susumu**
  **Kasugai-shi, Aichi 487-0032 (JP)**

(74) Representative: **Maxton Langmaack & Partner**
**Postfach 51 08 06**
**50944 Köln (DE)**

# (54) IONIC COMPOUND, COMPOSITION, CURED PRODUCT, HYDROGEL, AND OCULAR LENS

(57) An object of the invention is to provide an ionic compound capable of providing a cured material having superior antibacterial property and oxygen permeability, and a composition containing the ionic compound, and to provide a cured material, a hydrogel and an ophthalmic lens which have long-term sustainable antibacterial property and superior oxygen permeability. The present invention provides the ionic compound represented by the following formula (1):

$$\left[\left(P-Q^{+}\right)_{n} Z\right] \frac{n}{m} X^{m-} \qquad (1)$$

in the formula (1), P represents a polymerizable functional group. $Q^+$ represents a cationic group; Z represents an n-valent siloxanyl group in which the total atomic weight of a group of constituent atoms is no less than 70 and no greater than 1,000; $X^{m-}$ represents an m-valent anion; and n is an integer of 1 to 10, where provided that n is no less than 2, a plurality of Ps and $Q^+$s are each independently as defined above.

EP 2 581 394 A1

## Description

[TECHNICAL FIELD]

The present invention relates to an ionic compound suitable for ophthalmic lens materials, a composition containing the ionic compound, and a cured material, a hydrogel and an ophthalmic lens which are obtained from the composition.

[BACKGROUND ART]

Hydrogels obtained by swelling polymer compounds such as polyhydroxyethyl methacrylate with aqueous solvents are utilized in various fields including ophthalmic lenses such as contact lenses, since they have similar elasticity to living systems, and provide superior wearing sensation. Typical examples of the hydrogels used for the ophthalmic lenses include silicone hydrogels prepared by copolymerizing silicone monomers or macromers with hydrophilic monomers.

On the other hand, the ophthalmic lenses are directly worn on the eye, and thus must be always used under sanitary conditions. However, the ophthalmic lenses utilizing the hydrogels as described above are prone to adhesion of waste products in the lacrimal fluid and bacteria in the air, and to proliferation of the bacteria within the hydrogel, whereby eye diseases and the like may be induced. With respect to such a disadvantage, the cleanliness of the lenses can be kept by periodically subjecting the lenses to disinfection such as boiling. However, the disinfection treatment is cumbersome, and moreover the effect of the disinfection does not sustain over a prolonged period of time.

Furthermore, another method has been also proposed in which the contact lenses are immersed in a liquid for cleaning and storage of the contact lenses, referred to as a multipurpose solution, which includes sterilization components, and is widely used as a technique relating to antibacterial protection of the contact lenses. However, the method does eliminate the problem of cumbersomeness, but has a disadvantage that antibacterial components are washed away together with the lacrimal fluid during the wearing of the contact lens, and the effect of suppressing bacterial infection during the wearing of the contact lens is limited. Thus, techniques for imparting the antibacterial effect to the ophthalmic lens itself have been developed.

Examples of proposed contact lenses with antibacterial properties include (1) a contact lens having an antibacterial property provided by kneading antibacterial ceramics carrying a metal selected from silver, zinc and copper into a base material such as a hydrogel, or by coating the base material with the antibacterial ceramics (see, Japanese Unexamined Patent Application, Publication No. H4-76518), (2) a hydrogel containing a copolymer obtained from a hydrocarbon group-containing (meth)acrylates which includes a hydroxyl group and optionally has an intrachain ether linkage, and a monomer having a quaternary ammonium salt in its side chain, and a soft contact lens formed of the hydrogel (Japanese Unexamined Patent Application, Publication No. H6-337378), and the like.

However, with respect to the contact lens described in the above (1), since elution and/or detachment of the antibacterial ceramics and/or the carried metal are caused during the use of the contact lens over a prolonged period of time, the disadvantage of the impairment of the antibacterial property with time has never been solved. On the other hand, with respect to the contact lens in the above (2), the component with antibacterial property can be incorporated into the polymer chain as the monomer, the problem of the impairment of the antibacterial property with time due to the elution of the component is ameliorated. However, the contact lens described in (2) has a disadvantage that if the blend amount of the above-specified monomer is increased for the purpose of enhancing the antibacterial property, oxygen permeability of the resultant contact lens is deceased.

[PRIOR ART DOCUMENTS]

[PATENT DOCUMENTS]

Patent Document 1: Japanese Unexamined Patent Application, Publication No. H4-76518
Patent Document 2: Japanese Unexamined Patent Application, Publication No. H6-337378

[SUMMARY OF THE INVENTION]

[PROBLEMS TO BE SOLVED BY THE INVENTION]

The present invention was made in view of the foregoing background. An object of the invention is to provide

an ionic compound capable of providing a cured material having long-term sustainable antibacterial properties and superior oxygen permeability, and a composition containing the ionic compound, and to provide a cured material, a hydrogel and an ophthalmic lens which have long-term sustainable antibacterial properties and superior oxygen permeability.

[MEANS FOR SOLVING THE PROBLEMS]

In one aspect, the invention made for solving the foregoing problem provides:

an ionic compound represented by the following formula (1):

$$\left[\left(P-Q^{+}\right)_{n} Z\right] \ \frac{n}{m} X^{m-} \qquad (1)$$

in the formula (1), P represents a polymerizable functional group; $Q^+$ represents a cationic group; Z represents a siloxanyl group in which the total atomic weight of a group of constituent atoms is no less than 70 and no greater than 1,000; $X^{m-}$ represents an m-valent anion; and n is an integer of 1 to 10, wherein provided that n is no less than 2, a plurality of Ps and $Q^+$s are each independently as defined above.

Since the ionic compound has the cationic group ($Q^+$) and the anion ($X^{m-}$), a cured material having a superior antibacterial property can be obtained. Moreover, since the ionic compound has the polymerizable functional group (P), the ionic compound can be incorporated into a polymer chain, which may prevent impairment of the superior antibacterial property of the resultant cured material over time. Furthermore, since the siloxanyl group (Z) is included at the ends of the cationic moiety opposing to the polymerizable functional group (P) in the ionic compound, the resultant cured material can have the superior oxygen permeability even when the blend amount of the ionic compound is increased.

It is preferred that $P-Q^+-$ in the ionic compound is represented by the following formula (1-1), formula (1-2) or formula (1-3), and n is 1.

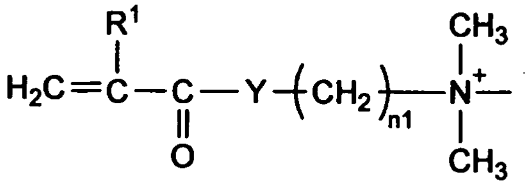

(1 - 1)

In the formula (1-1), $R^1$ represents a hydrogen atom or a methyl group; Y represents -O- or -NH-; and n1 is an integer of 1 to 18.

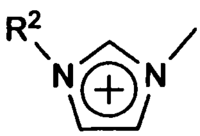

(1 - 2)

In the formula (1-2), $R^2$ represents $CH_2$=CH-, $CH_2$=CH-$CH_2$-, or a group represented by the following formula (2):

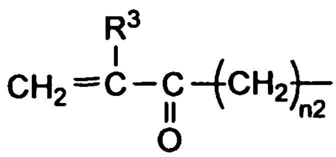

(2)

in the formula (2), $R^3$ represents a hydrogen atom or a methyl group; and n2 is an integer of 0 to 18.

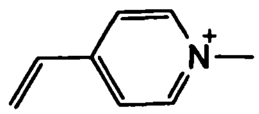

(1-3)

In the ionic compound, since the polymerizable functional group moiety and the cationic group moiety have the above-described structure, it is possible to efficiently obtain the cured material having still superior antibacterial property.

In the ionic compound, Z is preferably a group represented by the following formula (3).

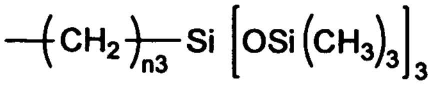

(3)

In the formula (3), n3 is an integer of 1 to 10.

The ionic compound has the siloxanyl group having the above-described structure at its end, and thereby enabling oxygen permeability of the resultant cured material to be further enhanced.

It is preferred that in the ionic compound, $X^{m-}$ contains a fluorine atom. According to the ionic compound, by using a fluorine atom-containing anion as the anion, hydrous characteristic of the resultant cured material can fall within a range suitable for ophthalmic lenses, for example. Therefore, comfortable wearing sensation can be imparted to the ophthalmic lens obtained by using the ionic compound.

It is preferred that $X^{m-}$ is at least one selected from the group consisting of the anions represented by the following formula (4) and formula (5).

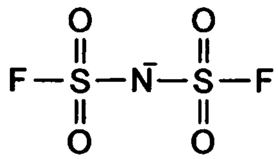

(4)

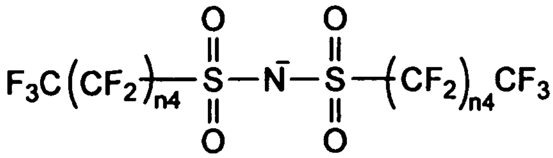

(5)

In the formula (5), n4 is an integer of 0 to 3.

According to the ionic compound, by using the anion of the above-described structure as the fluorine atom-containing anion, it is possible to allow the resultant cured material to possess moderate hydrous characteristics, while maintaining a high level of superior antibacterial property and oxygen permeability. Moreover, the ionic compound enables miscibility with other monomers to be improved in the case of attempts to produce its copolymers.

The composition according to an aspect of the present invention is a composition which contains the above-described ionic compound as the component (A). The composition enables a cured material which possesses the superior oxygen permeability and long-term sustainable antibacterial property to be obtained by curing the composition.

It is preferred that the composition further contains (B) a hydrophilic monomer, and (C) a silicone compound. Due to the composition further containing the components (B) and (C), the composition can cause the oxygen permeability and antibacterial property of the resultant cured material to be exhibited in a well-balanced manner, and can additionally impart moderate hydrophilicity to the resultant cured material. It should be noted that the hydrophilic monomer (B) and the silicone compound (C) shall not include the ionic compound (A). Furthermore, the hydrophilic monomer (B) shall not include the silicone compound (C).

The cured material according to another aspect of the present invention is a cured material obtained from the composition. Since the cured material contains a polymer having the ionic compound (A) incorporated into its polymer chain as the monomer, the cured material has the superior oxygen permeability and the long-term sustainable antibacterial property in combination, as described above.

The hydrogel according to yet another aspect of the present invention is a hydrogel which contains the cured material. Since the hydrogel contains the cured material which contains a polymer having the ionic compound (A) incorporated into its polymer chain as the monomer, the cured material has the superior oxygen permeability and the

long-term sustainable antibacterial property in combination, as described above.

The ophthalmic lens according to still another aspect of the present invention has the superior oxygen permeability and the long-term sustainable antibacterial property in combination, since the ophthalmic lens is formed of the hydrogel.

[EFFECTS OF THE INVENTION]

As explained in the foregoing, the ionic compound according to the aspect of present invention can maintain the superior antibacterial property over a long period of time, and enables a cured material having superior oxygen permeability to be obtained. Therefore, the cured material obtained from the composition containing the ionic compound can be widely used as a material having antibacterial properties and oxygen permeability, in ophthalmic lenses, materials for medical instruments and the like.

[DESCRIPTION OF EMBODIMENTS]

Hereinafter, embodiments of the present invention will be explained in the order of: an ionic compound, a composition containing the ionic compound, a cured material obtained from the composition, a hydrogel containing the cured material, and an ophthalmic lens formed of the hydrogel.

Ionic Compound

The ionic compound according to an embodiment of the present invention is represented by the above formula (1). The ionic compound has the cationic group ($Q^+$) and the anion ($X^{m-}$) , and thus can provide a cured material having a superior antibacterial property. Moreover, since the ionic compound has the polymerizable functional group (P), the ionic compound can be incorporated into a polymer chain, which may prevent the superior antibacterial property of the resultant cured material from impairing over time. Furthermore, since the siloxanyl group (Z) is included at the ends of the cationic moiety opposing to the polymerizable functional group (P) in the ionic compound, the resultant cured material can have the superior oxygen permeability.

The polymerizable functional group represented by P in the above formula (1) is not particularly limited, and typical examples thereof include ethylenic unsaturated groups. Specific examples of the ethylenic unsaturated groups include a vinyl group, an allyl group, a (meth)acryl group, an α-substituted acryl group, an ethylene group, and a styryl group. Of these, a (meth)acryl group is preferred in view of its polymerizablity.

The cationic group represented by $Q^+$ in the above formula (1) is a group having bond valence of 2 and positive charge of +1, and examples thereof include nitrogen atom-containing organic groups such as iminium ion structures, imidazolium ion structures, pyridinium ion structures, which are exemplified hereinbelow in sequence.

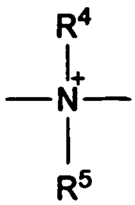

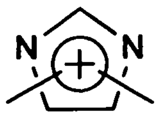

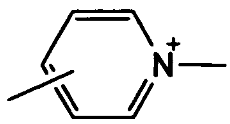

In the above formula, $R^4$ and $R^5$ each independently represent a hydrogen atom or an alkyl group having 1 to 3 carbon atoms.

Of these, the following groups are preferred in view of the ease of synthesis of the ionic compound.

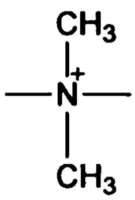

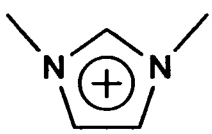

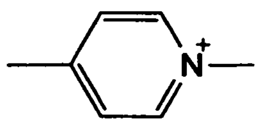

Z (siloxanyl group) in the above formula (1) is not particularly limited as long as the group contains at least one Si-O-Si bond and the total atomic weight of a group of atoms constituting the group is no less than 70 and no greater than 1,000; and examples of the siloxanyl group whose bond valence is 1 (n = 1) include a group represented by the following formula (6).

$$-\!\left(CH_2\right)_{n3}\!-Si\left[OSi\left(R^6\right)_3\right]_3 \qquad (6)$$

In the above formula (6), n3 is an integer of 1 to 10; and $R^6$ each independently represents an alkyl group, with the proviso that the total atomic weight of atoms constituting the group represented by the above formula (6) is no less than 70 and no greater than 1,000.

Of the groups represented by the above formula (6), the group in which all $R^6$'s represent a methyl group, i.e., the group represented by the above formula (3) is preferred in view of the ease of synthesis of the ionic compound according to the present invention. The ionic compound has the siloxanyl group of the above-described structure at one end, and thereby enabling oxygen permeability of the resultant polymer to be further enhanced. Similarly, n3 is preferably no less than 2 and no greater than 5 in view of the ease of synthesis of the ionic compound according to the present invention.

Preferred examples of $P\text{-}Q^+$- include a structure specifically depicted in the above formula (1-1), formula (1-2) or formula (1-3), in which n is 1.

According to the ionic compound, due to the polymerizable functional group moiety and the cationic group moiety having the above-described structure, it is possible to efficiently obtain a cured material having a superior antibacterial property.

In the above formula (1-1), n1 is preferably an integer of 1 to 10, and more preferably an integer of 1 to 5 in view of the ease of synthesis of the ionic compound according to the present invention.

Examples of the anion, $X^{m-}$, in the above formula (1) are not particularly limited, and include halide ions such as $Cl^-$, $I^-$, and $Br^-$, fluorine atom-containing anions, $NO_3^-$, $ClO_4^-$, $CH_3COO^-$, $SO_4^{2-}$ and the like. Of these, the fluorine atom-containing anions are preferred. According to the ionic compound, by using as the anion the fluorine atom-containing anions, hydrous characteristic of the resultant cured material can be kept in a range suitable, for example, for ophthalmic lenses. Therefore, comfortable wearing sensation can be imparted to the ophthalmic lenses obtained by using the ionic compound.

The fluorine atom-containing anion is exemplified by $F^-$, $BF_4^-$, $PF_6^-$, $AsF_6^-$, $SbF_6^-$, $(CF_3SO_2)_3C^-$, $(C_2F_5SO_2)_3C^-$, $(C_3F_7SO_2)_3C^-$, $(C_4F_9SO_2)_3C^-$, $F\,(HF)_n^-$, $CF_3SO_3^-$, $CF_3COO^-$, anions represented by the above formula (4) or formula (5), and the like.

Of these fluorine atom-containing anions, at least one selected from the group consisting of the anions represented by the above formula (4) and formula (5) are preferred. By using the anion of the above-described structure as the fluorine atom-containing anion, it is possible for the resultant cured material to possess an appropriate hydrous characteristic, while maintaining a high level of superior antibacterial property and oxygen permeability. Moreover, due to the ionic compound having the anion of the above structure, its miscibility with other monomers in the case of production of its copolymers can be improved.

In addition, n/m in the above formula (1) is the ratio of the number of the anion to the number of the cation in the ionic compound, and indicates that the ionic compound according to the present invention is electrically neutral. In other words, the cation with n-valent positive charge and the anion with m-valent negative charge are present in the ionic compound at the ratio of m:n. This n/m may be either an integer or a fraction.

Synthesis Method of Ionic Compound

The ionic compound may be synthesized, for example, by reacting a halogenated alkyl having a siloxanyl group (such as 3-iodopropyltris(trimethylsiloxy)silane) and an amine compound having a polymerizable functional group (dimethylaminoethyl acrylate) in a solvent. The solvent used herein is not particularly limited as long as the solvent is capable of dissolving each component, and may include toluene and the like.

Composition Containing Ionic Compound

The composition according to the present invention is a composition containing as the component (A), the ionic compound described above. In addition, the composition according to the present invention contains, as preferable components, the hydrophilic monomer (B) and the silicone compound (C), and may further contain other optional components. Hereinafter, each component will be explained.

[(A) Ionic Compound]

The ionic compound (A) contained in the composition is as described above, and detailed explanation thereabout is omitted here.

The percentage of the component (A) used in the composition is not particularly limited, and is preferably no less than 0.1% by mass and no greater than 30% by mass, and more preferably no less than 0.2% by mass and no greater than 20% by mass. By employing the percentage of the component (A) used of no less than 0.1% by mass, excellent antibacterial property can be achieved, whereas by employing the percentage of the component (A) of no greater than 30% by mass, the impairment of stability and transparency of the hydrogel can be suppressed.

[(B) Hydrophilic Monomer]

The hydrophilic monomer of the component (B) is not particularly limited as long as the hydrophilic monomer is capable of copolymerizing with the ionic compound having a polymerizable functional group of the component (A) and has a hydrophilic group. The hydrophilic monomer of the component (B) is exemplified by a monomer having a hydrophilic group and a polymerizable functional group, as well as a monomer having a hydrophilic and polymerizable functional group. Examples of the polymerizable functional group carried by the hydrophilic monomer include groups similar to those exemplified in A in the ionic compound described above.

Specific examples of the hydrophilic monomer include:

carboxylic acids such as (meth)acrylic acid, itaconic acid, crotonic acid, and vinylbenzoic acid;
(meth)acrylates having a hydroxyl group such as hydroxymethyl (meth)acrylate, 2-hydroxyethyl (meth)acrylate, 2-hydroxypropyl (meth)acrylate, 2,3-dihydroxypropyl (meth)acrylate, mono(meth)acrylate of polyethylene glycol, and mono(meth)acrylate of polypropylene glycol;
(meth)acrylamides such as (meth)acrylamide, N-methyl(meth)acrylamide, N-ethyl(meth)acrylamide, N-hydroxyethyl(meth)acrylamide, N,N-dimethyl(meth)acrylamide, N,N-diethyl(meth)acrylamide, and N-ethyl-N-aminoethyl (meth)acrylamide;
(alkyl)aminoalkyl acrylates such as 2-dimethylaminoethyl acrylate, and 2-butylaminoethyl acrylate;
pyrrolidones having a polymerizable functional group such as N-vinyl-3-methyl-2-pyrrolidone, N-vinyl-4-methyl-2-pyrrolidone, N-vinyl-5-methyl-2-pyrrolidone, N-vinyl-3-ethyl-2-pyrrolidone, N-vinyl-4,5-dimethyl-2-pyrrolidone, N-vinyl-5,5-dimethyl-2-pyrrolidone, N-vinyl-3,3,5-trimethyl-2-pyrrolidone, N-(meth)acrylolyl-2-pyrrolidone, N-(meth) acrylolyloxyethyl-2-pyrrolidone, 1-methyl-3-methylene-2-pyrrolidone, 1-ethyl-3-methylene-2-pyrrolidone, 1-methyl-5-methylene-2-pyrrolidone, 1-ethyl-5-methylene-2-pyrrolidone, 5-methyl-3-methylene-2-pyrrolidone, 5-ethyl-3-methylene-2-pyrrolidone, 1-n-propyl-3-methylene-2-pyrrolidone, 1-n-propyl-5-methylene-2-pyrrolidone, 1-i-propyl-3-methylene-2-pyrrolidone, 1-i-propyl-5-methylene-2-pyrrolidone, 1-n-butyl-3-methylene-2-pyrrolidone, and 1-t-butyl-3-methylene-2-pyrrolidone;
N-vinylpiperidones such as N-vinyl-2-piperidone, N-vinyl-3-methyl-2-piperidone, N-vinyl-4-methyl-2-piperidone, N-vinyl-5-methyl-2-piperidone, N-vinyl-6-methyl-2-piperidone, N-vinyl-6-ethyl-2-piperidone, N-vinyl-3,5-dimethyl-2-piperidone, and N-vinyl-4,4-dimethyl-2-piperidone;
N-vinyllactams such as N-vinyl-2-caprolactam, N-vinyl-3-methyl-2-caprolactam, N-vinyl-4-methyl-2-caprolactam, N-vinyl-7-methyl-2-caprolactam, N-vinyl-7-ethyl-2-caprolactam, N-vinyl-3,5-dimethyl-2-caprolactam, N-vinyl-4,6-dimethyl-2-caprolactam, and N-vinyl-3,5,7-trimethyl-2-caprolactam;
N-vinylamides such as N-vinylformamide, N-vinyl-N-methylformamide, N-vinyl-N-ethylformamide, N-vinylacetamide, N-vinyl-N-methylacetamide, and N-vinyl-N-ethylacetamide;
other hydrophilic compounds having a polymerizable functional group such as aminostyrene, hydroxystyrene, vinyl acetate, glycidyl acrylate, allyl glycidyl ether, vinyl propionate, N-vinylimidazole, N-vinylpiperidine, N-vinylsuccinimide, N-vinylphthalimide, N-(meth)acryloylpiperidine, and N-(meth)acryloylmorpholine; and the like.

Of these hydrophilic monomers, (meth)acrylates having a hydroxyl group and (meth)acrylamides are preferred in view of mechanical characteristic and storage stability of the resultant hydrogel. Moreover, if the silicone compounds as described later are used, pyrrolidone derivatives in which the polymerizable group is a methylene group, and alkyl-substituted (meth)acrylamides are preferred in view of improving the miscibility with the silicone compound in the composition as well as imparting water wettability, lubricating property and ready wettability to a surface of the resultant lenses and the like. The hydrophilic monomers having a polymerizable functional group may be used alone, or in admixtures of two or more thereof. In addition, in the system using the components which undergoes hydrolysis by acids or bases, such as vinyl acetate, it is possible to impart further flexibility and water wettability to the ophthalmic lenses by treating the

ophthalmic lenses and the like with the acid and/or the base after their production.

The percentage of the component (B) used in the composition is not particularly limited, but is preferably no less than 30% by mass and no greater than 95% by mass, and more preferably no less than 40% by mass and no greater than 90% by mass. By employing the percentage of the component (B) used of no less than 30% by mass, a hydrogel having sufficient hydrophilicity and stability can be obtained. By employing the percentage of the component (B) of no greater than 95% by mass, the impairment of the antibacterial property and the like by the component (A) can suppressed.

[(C) Silicone Compound]

In order to impart high oxygen permeability and flexibility to the resultant cured material, a silicone compound may be further added into the composition as the component (C). Such a silicone compound is not particularly limited as long as it has a siloxanyl group. In addition, the silicone compound may have a polymerizable functional group, and examples of the polymerizable functional group include those similar to the examples for the components (A) and (B).

Examples of the silicone compound of the component (C) include those represented by the following formula (7).

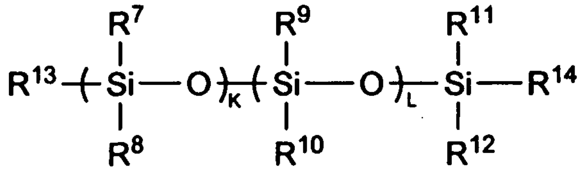

(7)

In the formula (7), $R^7$, $R^8$, $R^9$, $R^{10}$, $R^{11}$, $R^{12}$, $R^{13}$ and $R^{14}$ each independently represent an alkyl group having 1 to 6 carbon atoms, a fluorine-substituted alkyl group, an alkyl group having at least one amino group, an alkyl group having at least one hydroxyl group, an alkyl group having at least one epoxy group, an alkyl group having at least one carboxyl group, a phenyl group or a hydrogen atom; K is an integer of 10 to 100; L is an integer of 0 to 90; and K+L is an integer of 10 to 100.

Of the silicone compounds of the component (C), preferred examples of the compound having a polymerizable functional group include a compound having an ethylenic unsaturated group and a polydimethylsiloxane structure via a urethane linkage. Due to the silicone compound having the urethane linkage and the siloxane moiety, the silicone compound has the effect of imparting flexibility, elastic resilience, and oxygen permeability to the obtained cured materials such as lenses and the like, while improving their mechanical strength. In other words, since the silicone compound has an ethylenic unsaturated group, which is a polymerizable group, at both ends of the molecule, and is copolymerized with other copolymerization components via the polymerizable group, the silicone compound enables further improvement of the mechanical strength of the resultant cured materials through crosslinking of the molecule.

Typical examples of the compound having an ethylenic unsaturated group and a polydimethylsiloxane structure via a urethane linkage include a polysiloxane macromonomer represented by the following formula (i).

$A^1-U^1-(S^1-U^2)_n-S^2-U^3-A^2$ (i)

In the formula (i), $A^1$ represents a group represented by the general formula (ii):

$Y^{21}-Z^{21}-R^{21}-$ (ii)

In the formula (ii), $Y^{21}$ represents a (meth)acryloyl group, a vinyl group or an allyl group; $Z^{21}$ represents an oxygen atom or a direct bond; and $R^{21}$ represents a single bond or a linear, branched or aromatic-ring-bearing alkylene group having 1 to 12 carbon atoms.

$A^2$ represents a group represented by the general formula (iii):

$-R^{22}-Z^{22}-Y^{22}$ (iii)

In the formula (iii), $Y^{22}$ represents a (meth)acryloyl group, a vinyl group or an allyl group; $Z^{22}$ represents an oxygen atom or a direct bond; and $R^{22}$ represents a direct bond or a linear, branched or aromatic-ring-bearing alkylene group having 1 to 12 carbon atoms, in which $Y^{21}$ in the general formula (ii) and $Y^{22}$ in the general formula (iii) may be the same or different.

$U^1$ represents a group represented by the general formula (iv):

$-X^{21}-E^{21}-X^{25}-R^{23}-$ (iv)

In the formula (iv), $X^{21}$ and $X^{25}$ each independently represent at least one selected from the group consisting of a direct bond, an oxygen atom and an alkylene glycol group; $E^{21}$ represents -NHCO- group (in which $X^{21}$ represents a direct bond; $X^{25}$ represents an oxygen atom or an alkylene glycol group; and $E^{21}$ forms a urethane linkage with $X^{25}$), -CONH- group (in which $X^{21}$ represents an oxygen atom or an alkylene glycol group; $X^{25}$ represents a direct bond; and $E^{21}$ forms a urethane linkage with $X^{21}$) or a bivalent group derived from an diisocyanate selected from the group consisting of saturated aliphatic diisocyanates, unsaturated aliphatic diisocyanates, alicyclic diisocyanates and aromatic diisocyanates (in which $X^{21}$ and $X^{25}$ each independently represent at least one selected from an oxygen atom and an alkylene glycol group; and $E^{21}$ forms two urethane linkages with $X^{21}$ and $X^{25}$, respectively); and $R^{23}$ represents a linear or branched alkylene group having 1 to 6 carbon atoms.

$S^1$ and $S^2$ represents each independently a group represented by the general formula (v):

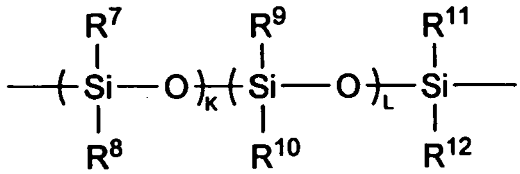

(v)

In the formula (v), $R^7$, $R^8$, $R^9$, $R^{10}$, $R^{11}$ and $R^{12}$ each independently represent an alkyl group having 1 to 6 carbon atoms, a fluorine-substituted alkyl group, a phenyl group or a hydrogen atom; K is an integer of 10 to 100; L is 0 or an integer of 1 to 90; and K+L is an integer of 10 to 100.

$U^2$ represents a group represented by the general formula (vi):

$-R^{24}-X^{27}-E^{24}-X^{28}-R^{25}-$ (vi)

In the formula (vi), $R^{24}$ and $R^{25}$ each independently represent a linear or branched alkylene group having 1 to 6 carbon atoms; $X^{27}$ and $X^{28}$ each independently represent an oxygen atom or an alkylene glycol group; and $E^{24}$ represents a bivalent group derived from a diisocyanate selected from the group consisting of saturated aliphatic diisocyanates, unsaturated aliphatic diisocyanates, alicyclic diisocyanates and aromatic diisocyanates, in which $E^{24}$ forms two urethane linkages with $X^{27}$ and $X^{28}$, respectively.

$U^3$ represents a group represented by the general formula (vii):

$-R^{26}-X^{26}-E^{22}-X^{22}-$ (vii)

In the formula (vii), $R^{26}$ represents a linear or branched alkylene group having 1 to 6 carbon atoms; $X^{22}$ and $X^{26}$ each independently represent at least one selected from the group consisting of a direct bond, an oxygen atom and an alkylene glycol group; $E^{22}$ represents -NHCO- group (in which $X^{22}$ represents an oxygen atom or an alkylene glycol group; $X^{26}$ represents a direct bond; and $E^{22}$ forms a urethane linkage with $X^{22}$), - CONH- group (in which $X^{22}$ represents a direct bond; $X^{26}$ represents an oxygen atom or an alkylene glycol group; and $E^{22}$ forms a urethane linkage with $X^{26}$) or a bivalent group derived from an diisocyanate selected from the group consisting of saturated aliphatic diisocyanates, unsaturated aliphatic diisocyanates, alicyclic diisocyanates and aromatic diisocyanates (in which $X^{22}$ and $X^{26}$ each independently represents at least one selected from an oxygen atom and an alkylene glycol group; and $E^{22}$ forms two urethane linkages with $X^{22}$ and $X^{26}$, respectively).

n is an integer of 0 to 10.

In the above general formula (i), each of $Y^{21}$ and $Y^{22}$ are a polymerizable group, and an acryloyl group is particularly preferred in view of its readiness for copolymerization with the hydrophilic monomer of the component (B).

In the above general formula (i), $Z^{21}$ and $Z^{22}$ each represent an oxygen atom or a direct bond, and preferably represent an oxygen atom. $R^{21}$ and $R^{22}$ each represent a direct bond or a linear, branched or aromatic-ring-bearing alkylene group having 1 to 12 carbon atoms, preferably an alkylene group having 2 to 4 carbon atoms. $U^1$, $U^2$ and $U^3$ represent the group that contains the urethane linkage in the molecular chain.

In $U^1$ and $U^3$ in the above general formula (i), $E^{21}$ and $E^{22}$ each represent, as described above, -CONH- group, -NHCO- group or a bivalent group derived from an diisocyanate selected from the group consisting of saturated aliphatic diisocyanates, unsaturated aliphatic diisocyanates, alicyclic diisocyanates and aromatic diisocyanates. Herein, examples of the bivalent group derived from an diisocyanate selected from the group consisting of saturated aliphatic diisocyanates, unsaturated aliphatic diisocyanates, alicyclic diisocyanates and aromatic diisocyanates include a bivalent group derived

from saturated aliphatic diisocyanates such as ethylene diisocyanate, 1,3-diisocyanatepropane, and hexamethylenediisocyanate; a bivalent group derived from alicyclic diisocyanates such as 1,2-diisocyanatecyclohexane, bis(4-isocyanatecyclohexyl)methane, and isophoronediisocyanate; a bivalent group derived from aromatic diisocyanates such as tolylenediisocyanate, and 1,5-diisocyanatenaphthalene; a bivalent group derived from unsaturated aliphatic diisocyanates such as 2,2'-diisocyanatediethyl fumarate. Of these examples, a bivalent group derived from hexamethylenediisocyanate, a bivalent group derived from tolylenediisocyanate and a bivalent group derived from isophoronediisocyanate are preferred in view of their availability and capability of imparting strength to the lenses.

In $U^2$ in the above general formula (1), $E^{24}$ represents, as described above, a bivalent group derived from a diisocyanate selected from the group consisting of saturated aliphatic diisocyanates, unsaturated aliphatic diisocyanates, alicyclic diisocyanates and aromatic diisocyanates. Herein, examples of the bivalent group derived from a diisocyanate selected from the group consisting of saturated aliphatic diisocyanates, unsaturated aliphatic diisocyanates, alicyclic diisocyanates and aromatic diisocyanates include bivalent groups similar to those in $U^1$ and $U^3$. Of these examples, a bivalent group derived from hexamethylenediisocyanate, a bivalent group derived from tolylenediisocyanate and a bivalent group derived from isophoronediisocyanate are preferred in view of their availability and capability of imparting strength to the lenses. Furthermore, $E^{24}$ forms two urethane linkages with $X^{27}$ and $X^{28}$, respectively. $X^{27}$ and $X^{28}$ preferably each independently represent an oxygen atom or an alkylene glycol group having 1 to 6 carbon atoms, and $R^{24}$ and $R^{25}$ represents each independently a linear or branched alkylene group having 1 to 6 carbon atoms.

$X^{21}$, $X^{25}$, $X^{27}$, $X^{28}$, $X^{22}$ and $X^{26}$ as above are exemplified by an alkylene glycol having 1 to 20 carbon atoms. Such an alkylene glycol having 1 to 20 carbon atoms is represented by the following general formula (viii).

$$-O-(C_xH_{2x}-O)_y- \quad (viii)$$

In the formula (viii), x is an integer of 1 to 4, and y is an integer of 1 to 5.

When $R^7$, $R^8$, $R^9$, $R^{10}$, $R^{11}$ and $R^{12}$ as above are a fluorine-substituted alkyl group, examples thereof include groups represented by $-(CH_2)_m-C_pF_{2p+1}$ (m = 1 to 10, p = 1 to 10). Specific examples of the fluorine-substituted alkyl group include linear fluorine-substituted alkyl groups such as a 3,3,3-trifluoro-n-propyl group, a 2-(perfluorobutyl)ethyl group and a 2-(perfluorooctyl)ethyl group; and branched fluorine-substituted alkyl groups such as a 2-(perfluoro-5-methylhexyl)ethyl group. When the blend amount of the compound having the fluorine-substituted alkyl group is increased, lipid-deposit resistance of the resultant cured material may be improved.

In the above general formula (v) representing $S^1$ and $S^2$, K is an integer of 10 to 100; L is 0 or an integer of 1 to 90; and the sum of K and L (i.e., K+L) is an integer of 10 to 100, preferably 10 to 80. If (K+L) is greater than 100, then the molecular weight of the silicone compound becomes high, whereby the miscibility of the silicone compound with the hydrophilic monomer of the component (B) is reduced, which causes the polymerization system to exhibit phase separation and white turbidity during the polymerization, and consequently homogeneous and transparent lenses and the like may not be obtained. Furthermore, if (K+L) is less than 10, then there is a tendency that the oxygen permeability of the resultant lenses and the like is lowered and their flexibility is also reduced.

In the above general formula (i), n is an integer of 0 to 10, preferably an integer of 0 to 5. If n is greater than 10, then the molecular weight of the silicone compound becomes high, whereby the miscibility of the silicone compound with the hydrophilic monomer of the component (B) is reduced, which causes the polymerization system to exhibit phase separation and white turbidity during the polymerization, and consequently homogeneous and transparent cured materials such as lenses and the like may not be obtained, as described above.

Typical examples of the silicone compound of the component (C) represented by the above general formula (i) include compounds represented by the following general formulae (C-1) and (C-2).

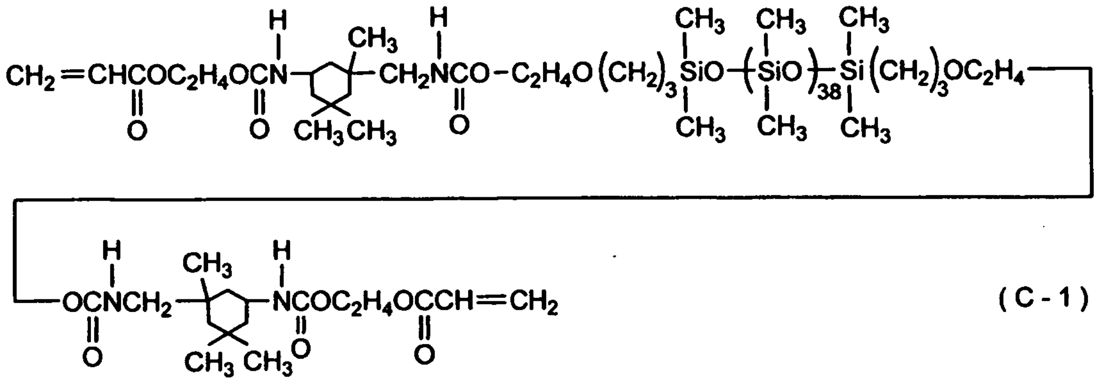

(C-1)

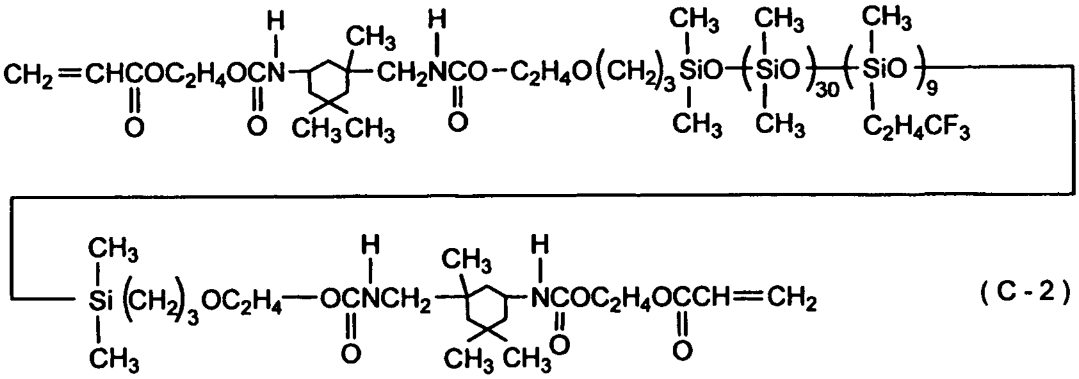

(C-2)

The silicone compound of the component (C) may have a hydrophilic partial structure in the molecule, irrespective of the presence or absence of the polymerizable functional group. Due to the silicone compound having the hydrophilic partial structure as described above, the miscibility of the silicone compound and the hydrophilic monomer of the component (B) can be improved, whereby the water wettability of the resultant lens can be improved. Examples of the hydrophilic partial structure of the silicone compound include polyethylene glycol, polypropylene glycol, polyvinyl alcohol, polyvinylpyrrolidone, poly(meth)acrylic acid, poly(meth)acrylic acid salts, poly(2-hydroxyethyl (meth)acrylate), polytetrahydrofuran, polyoxetane, polyoxazoline, polyacrylamide, polydimethylacrylamide, polydiethylacrylamide, poly(2-methacryloyloxyethylphosphorylcholine), block polymers thereof, and the like. The hydrophilic partial structure may be attached to the silicone compound in a comb-like fashion, or attached to either or both ends of the silicone compound. Total atomic weight of the atoms constituting the hydrophilic partial structure is preferably 100 to 1,000,000, and more preferably 1,000 to 500,000. If the molecular weight is less than 100, then the silicone compound may fail to impart sufficient hydrophilicity for achieving miscibility of the silicone compound with the hydrophilic monomer of the component (B). On the other hand, if the molecular weight is greater than 1,000,000, too large respective hydrophilic and hydrophobic domains tends to render it difficult to yield transparent cured materials.

Examples of the silicone compound having the hydrophilic partial structure include a compound represented by the general formula (8).

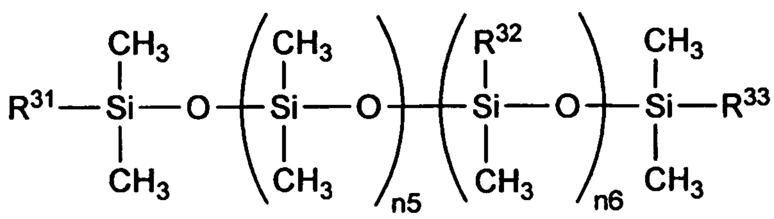

(8)

In the formula (8), $R^{31}$, $R^{32}$ and $R^{33}$ each independently represent an alkyl group having 1 to 6 carbon atoms, a hydrogen atom or a group represented by the following general formula (9); n5 is an integer of 5 to 100; n6 is an integer of 0 to

100; and at least one of $R^{31}$, $R^{32}$ and $R^{33}$ represent a group represented by the general formula (9).

$$-\left(CH_2\right)_3-O-\left(CH_2CH_2O\right)_{n7}R^{34} \qquad (9)$$

In the formula (9), n7 is an integer of 1 to 100; and $R^{34}$ represents an alkyl group having 1 to 22 carbon atoms or a hydrogen atom.

In order to further improve the oxygen permeability of the resultant cured material used for ophthalmic lenses and the like, and to impart flexibility thereto, the composition may contain other silicone compounds than those represented by the above general formula (i). Examples of such other silicone compounds include silicone-containing alkyl (meth) acrylates, silicone-containing styrene derivatives, silicone-containing fumaric acid diesters, and the like.

Examples of the silicone-containing alkyl (meth)acrylates include trimethylsiloxydimethylsilylmethyl (meth)acrylate, trimethylsiloxydimethylsilylpropyl (meth)acrylate, methylbis(trimethylsiloxy)silylpropyl (meth)acrylate, tris(trimethylsiloxy)silylpropyl (meth)acrylate, mono[methylbis(trimethylsiloxy)siloxy]bis(trimethylsiloxy)silylpropyl (meth)acrylate, tris[methylbis(trimethylsiloxy)siloxy]silylpropyl (meth)acrylate, methylbis(trimethylsiloxy)silylpropylglyceryl (meth)acrylate, tris(trimethylsiloxy)silylpropylglyceryl (meth)acrylate, mono[methylbis(trimethylsiloxy)siloxy]bis(trimethylsiloxy)silylpropylglyc eryl (meth)acrylate, trimethylsilylethyltetramethyldisiloxypropylglyceryl (meth)acrylate, trimethylsilylmethyl (meth)acrylate, trimethylsilylpropyl (meth)acrylate, trimethylsilylpropylglyceryl (meth)acrylate, trimethylsiloxydimethylsilylpropylglyceryl (meth)acrylate, methylbis(trimethylsiloxy)silylethyltetramethyldisiloxymethyl (meth)acrylate, tetramethyltriisopropylcyclotetrasiloxanylpropyl (meth)acrylate, tetramethyltriisopropylcyclotetrasiloxybis(trimethylsiloxy)silylpropyl (meth)acrylate, and the like.

Examples of the silicone-containing styrene derivative include a compound represented by the following general formula (10), and the like.

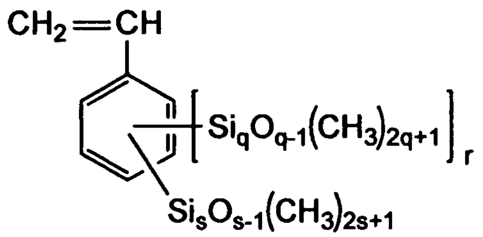

(10)

In the formula (10), q is an integer of 1 to 15; r represents 0 or 1; and s is an integer of 1 to 15.

Specific examples of the silicone-containing styrene derivatives represented by the above general formula (10) include tris(trimethylsiloxy)silylstyrene, bis(trimethylsiloxy)methylsilylstyrene, (trimethylsiloxy)dimethylsilylstyrene, tris (trimethylsiloxy)siloxydimethylsilylstyrene, [bis(trimethylsiloxy)methylsiloxy]dimethylsilylstyrene, (trimethylsiloxy)dimethylsilylstyrene, heptamethyltrisiloxanylstyrene, nonamethyltetrasiloxanylstyrene, pentadecamethylheptasiloxanylstyrene, heneicosamethyldecasiloxanylstyrene, heptacosamethyltridecasiloxanylstyrene, hentriacontamethylpentadecasiloxanylstyrene, trimethylsiloxypentamethyldisiloxymethylsilylstyrene, tris(pentamethyldisiloxy)silylstyrene, tris(trimethylsiloxy)siloxybis(trimethylsiloxy)silylstyrene, bis(heptamethyltrisiloxy)methylsilylstyrene, tris[methylbis(trimethylsiloxy)siloxy]silylstyrene, trimethylsiloxybis[tris(trimethylsiloxy)siloxy]silylstyrene, heptakis(trimethylsiloxy)trisilylstyrene, nonamethyltetrasiloxyundecylmethylpentasiloxymethylsilylstyrene, tris[tris(trimethylsiloxy)siloxy]silylstyrene, (tristrimethylsiloxyhexamethyl)tetrasiloxy[tris(trimethylsiloxy)siloxy]trimethylsiloxysilylstyrene, nonakis(trimethylsiloxy)tetrasilylstyrene, bis(tridecamethylhexasiloxy)methylsilylstyrene, heptamethylcyclotetrasiloxanylstyrene, heptamethylcyclotetrasiloxybis(trimethylsiloxy)silylstyrene, tripropyltetramethylcyclotetrasiloxanylstyrene, trimethylsilylstyrene, and the like.

Examples of the silicone-containing fumaric acid diesters include a compound represented by the following general formula (11), and the like.

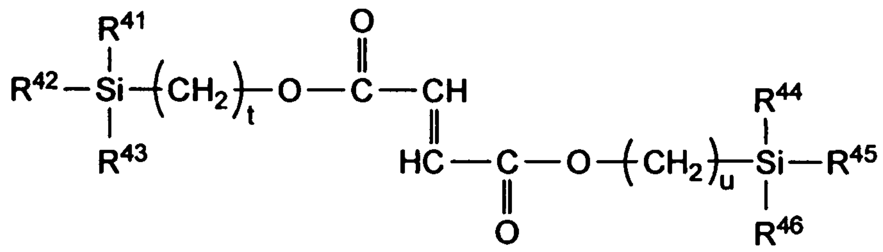

(11)

In the formula (11), $R^{41}$, $R^{42}$, $R^{43}$, $R^{44}$, $R^{46}$ and $R^{47}$ each independently represent a methyl group or a trimethylsiloxy group.

Specific examples of the silicone-containing fumaric acid diesters represented by the above general formula (11) include bis[3-(trimethylsilyl)propyl] fumarate, bis[3-(pentamethyldisiloxanyl)propyl] fumarate, bis[tris(trimethylsiloxy)silylpropyl] fumarate, and the like.

The percentage of the component (C) used in the composition is not particularly limited, and is preferably no less than 10% by mass and no greater than 80% by mass, and more preferably no less than 20% by mass and no greater than 70% by mass. By employing the percentage of the component (C) used of no less than 10% by mass, it is possible to obtain hydrogels with sufficient oxygen permeability and high flexibility. By employing the percentage of the component (C) of no greater than 80% by mass, the impairment of hydrophilicity, transparency and the like of the resultant cured material may be prevented.

((D) Other Polymerizable Component)

If it is intended to further impart desired characteristics to the resultant cured material such as the ophthalmic lens, (D) other polymerizable component such as alkyl (meth)acrylates, fluorine-containing alkyl (meth)acrylates, hardness-modifying monomers, polymerizable ultraviolet ray absorbing agents, polymerizable coloring matters, polymerizable ultraviolet ray absorbing coloring matters may be used.

(Alkyl (Meth)acrylate)

The alkyl (meth)acrylate is added to the composition for the purpose of modifying hardness of the ophthalmic lens and the like to impart rigidity or softness. Examples of the alkyl (meth)acrylate include (meth)acrylates of a linear, branched or cyclic alkyl such as methyl (meth)acrylate, ethyl (meth)acrylate, isopropyl (meth)acrylate, n-propyl (meth)acrylate, isobutyl (meth)acrylate, n-butyl (meth)acrylate, 2-ethylhexyl (meth)acrylate, n-octyl (meth)acrylate, n-decyl (meth)acrylate, n-dodecyl (meth)acrylate, t-butyl (meth)acrylate, pentyl (meth)acrylate, t-pentyl (meth)acrylate, hexyl (meth)acrylate, heptyl (meth)acrylate, nonyl (meth)acrylate, stearyl (meth)acrylate, cyclopentyl (meth)acrylate, and cyclohexyl (meth)acrylate. These alkyl (meth)acrylates may be used alone, or in admixtures of two or more thereof.

(Fluorine-Containing Alkyl (Meth)acrylate)

The fluorine-containing alkyl (meth)acrylate is added to the composition for the purpose of improving lipid-deposit resistance of the ophthalmic lens and the like. Examples of the fluorine-containing alkyl (meth)acrylates include a compound represented by the following general formula (12), and the like:

$$CH_2=CR^{51}COOC_vH_{(2v-w+1)}F_w \quad (12)$$

In the formula (12), $R^{51}$ represents a hydrogen atom or $CH_3$; v is an integer of 1 to 15, and w is an integer of 1 to (2v+1).

Specific examples of the compound represented by the above general formula (12) include 2,2,2-trifluoroethyl (meth)acrylate, 2,2,3,3-tetrafluoropropyl (meth)acrylate, 2,2,3,3-tetrafluoro-t-pentyl (meth)acrylate, 2,2,3,4,4,4-hexafluorobutyl (meth)acrylate, 2,2,3,4,4,4-hexafluoro-t-hexyl (meth)acrylate, 2,3,4,5,5,5-hexafluoro-2,4-bis(trifluoromethyl)pentyl (meth)acrylate, 2,2,3,3,4,4-hexafluorobutyl (meth)acrylate, 2,2,2,2',2',2'-hexafluoroisopropyl (meth)acrylate, 2,2,3,3,4,4,4-heptafluorobutyl (meth)acrylate, 2,2,3,3,4,4,5,5-octafluoropentyl (meth)acrylate, 2,2,3,3,4,4,5,5,5-nonafluoropentyl (meth)acrylate, 2,2,3,3,4,4,5,5,6,6,7,7-dodecafluoroheptyl (meth)acrylate, 3,3,4,4,5,5,6,6,7,7,8,8-dodecafluorooctyl (meth)acrylate, 3,3,4,4,5,5,6,6,7,7,8,8,8-tridecafluorooctyl (meth)acrylate, 2,2,3,3,4,4,5,5,6,6,7,7,7-tridecafluoroheptyl (meth)acrylate, 3,3,4,4,5,5,6,6,7,7,8,8,9,9,10,10-hexadecafluorodecyl (meth)acrylate, 3,3,4,4,5,5,6,6,7,7,8,8,9,9,10,10,10-heptadecafluorodecyl (meth)acrylate, 3,3,4,4,5,5,6,6,7,7,8,8,9,9,10,10,11,11-octadecafluoroundecyl (meth)acrylate, 3,3,4,4,5,5,6,6,7,7,8,8,9,9,10,10,11,11,11-nonadecafluoroundecyl (meth)acrylate, 3,3,4,4,5,5,6,6,7,7,8,8,9,9,10,10,11,11,12,12-eicosafluorododecyl (meth)acrylate and the like. These fluorine-containing alkyl

(meth)acrylates may be used alone, or in admixtures of two or more thereof.

The percentage of the alkyl (meth)acrylate and the fluorine-containing alkyl (meth)acrylate used in the composition may be preferably no less than 0.01% by mass and no greater than 20% by mass, and more preferably no less than 0.1% by mass and no greater than 10% by mass. By employing the percentage of the alkyl (meth)acrylate and the fluorine-containing alkyl (meth)acrylate used of no less than 0.01% by mass, it is possible to appropriately modify hardness of the ophthalmic lens or improve their lipid-deposit resistance. On the other hand, by employing the percentage of the alkyl (meth)acrylate and the fluorine-containing alkyl (meth)acrylate of no greater than 20% by mass, the effect of the antibacterial property, transparency, oxygen permeability and the like provided by the other components may be sufficiently exhibited.

(Hardness-Modifying Monomer)

The hardness-modifying monomer is added to the composition for the purpose of modifying the hardness of the cured material such as the ophthalmic lens to impart rigidity or softness. Examples of the hardness-modifying monomer include alkoxyalkyl (meth)acrylates such as 2-ethoxyethyl (meth)acrylate, 3-ethoxypropyl (meth)acrylate, 2-methoxyethyl (meth)acrylate, and 3-methoxypropyl (meth)acrylate; alkylthioalkyl (meth)acrylates such as ethylthioethyl (meth)acrylate, and methylthioethyl (meth)acrylate; and styrenes such as styrene, α-methylstyrene, methylstyrene, ethylstyrene, propylstyrene, butylstyrene, t-butylstyrene, isobutylstyrene, pentylstyrene, methyl-α-methylstyrene, ethyl-α-methylstyrene, propyl-α-methylstyrene, butyl-α-methylstyrene, t-butyl-α-methylstyrene, isobutyl-α-methylstyrene, and pentyl-α-methylstyrene. These hardness-modifying monomers may be used alone, or in admixtures of two or more thereof.

The percentage of the hardness-modifying monomer used in the composition may be preferably no less than 1% by mass and no greater than 30% by mass, and more preferably no less than 3% by mass and no greater than 20% by mass. By employing the percentage of the hardness-modifying monomers used of no less than 1% by mass, it is possible to sufficiently impart desired rigidity or softness to the ophthalmic lens and the like. On the other hand, by employing the percentage of the hardness-modifying monomers of no greater than 30% by mass, the impairment of the oxygen permeability and mechanical strength of the ophthalmic lens and the like may be suppressed.

(Polymerizable Ultraviolet Ray Absorbing Agent)

The polymerizable ultraviolet ray absorbing agent is added to the composition for the purpose of imparting ultraviolet ray absorptivity to the ophthalmic lens and the like.

Examples of the polymerizable ultraviolet ray absorbing agent include benzophenone-based polymerizable ultraviolet ray absorbing agents such as 2-hydroxy-4-(meth)acryloyloxybenzophenone, 2-hydroxy-4-(meth)acryloyloxy-5-t-butylbenzophenone, 2-hydroxy-4-(meth)acryloyloxy-2',4'-dichlorobenzophenone, and 2-hydroxy-4-(2'-hydroxy-3'-(meth)acryloyloxypropoxy)benzophenone; benzotriazole-based polymerizable ultraviolet ray absorbing agents such as 2-(2'-hydroxy-5'-(meth)acryloyloxyethylphenyl)-2H-benzotriazole, 2-(2'-hydroxy-5'-(meth)acryloyloxyethylphenyl)-5-chloro-2H-benzotriazole, 2-[3-(2H-benzotriazol-2-yl)-4-hydroxyphenyl]ethyl methacrylate, 2-(2'-hydroxy-5'-(meth)acryloyloxypropylphenyl)-2H-benzotriazole, 2-(2'-hydroxy-5'-(meth)acryloyloxypropyl-3'-t-butylphenyl)-5-chloro-2H-benzotriazole, 2-(2'-hydroxy-5'-(2"-methacryloyloxyethoxy)-3'-t-butylphenyl)-5-methyl-2H-benzotriazole, and 2-[2'-hydroxy-5'-(2-methacryloyloxyethyl)phenyl]-2H-benzotriazole; salicylic acid derivative-based polymerizable ultraviolet ray absorbing agents such as phenyl 2-hydroxy-4-methacryloyloxymethylbenzoate; and 2-cyano-3-phenyl-3-(3'-(meth)acryloyloxyphenyl)propenoic acid methyl ester, and the like. These polymerizable ultraviolet ray absorbing agents may be used alone, or in admixtures of two or more thereof.

(Polymerizable Coloring Matter)

The polymerizable coloring matter is added to the composition for the purpose of coloring the ophthalmic lens and the like.

Examples of the polymerizable coloring matters include: azo-based polymerizable coloring matters such as 1-phenylazo-4-(meth)acryloyloxynaphthalene, 1-phenylazo-2-hydroxy-3-(meth)acryloyloxynaphthalene, 1-naphthylazo-2-hydroxy-3-(meth)acryloyloxynaphthalene, 1-(α-anthrylazo)-2-hydroxy-3-(meth)acryloyloxynaphthalene, 1-((4'-(phenylazo)-phenyl)azo)-2-hydroxy-3-(meth)acryloyloxynaphthalene, 1-(2',4'-xylylazo)-2-(meth)acryloyloxynaphthalene, 1-(o-tolylazo)-2-(meth)acryloyloxynaphthalene, 2-(m-(meth)acryloylamido-anilino)-4,6-bis(1'-(o-tolylazo)-2'-naphthylamino)-1,3,5-triazine, 2-(m-vinylanilino)-4-(4'-nitrophenylazoanilino)-6-chloro-1,3,5-triazine, 2-(1'-(o-tolylazo)-2'-naphthyloxy)-4-(m-vinylanilino)-6-chloro-1,3,5-triazine, 2-(p-vinylanilino)-4-(1'-(o-tolylazo)-2'-naphthylamino)-6-chloro-1,3,5-triazine, N-(1'-(o-tolylazo)-2'-naphthyl)-3-vinylphthalic acid monoamide, N-(1'-(o-tolylazo)-2'-naphthyl)-6-vinylphthalic acid monoamide, 3-vinylphthalic acid-(4'-(p-sulfophenylazo)-1'-naphthyl)mono ester, 6-vinylphthalic acid-(4'-(p-sulfophenylazo)-1'-naphthyl)mono ester, 3-(meth)acryloylamido-4-phenylazo phenol, 3-(meth)acryloylamido-4-(8'-hydroxy-3',6'-disulfo-1'-naphthylazo)-phenol, 3-(meth)acryloylamido-4-(1'-phenylazo-2'-naphthylazo)-phenol, 3-(meth) acryloylamido-4-(p-tolylazo)phenol, 2-amino-4-(m-(2'-hydroxy-1'-naphthylazo)anilino)-6-isopropenyl-1,3,5-triazine, 2-amino-4-(N-methyl-p-(2'-hydroxy-1'-naphthylazo)anilino)-6-isopropenyl-1,3,5-triazine, 2-amino-4-(m-(4'-hydroxy-1'-phenylazo)anilino)-6-isopropenyl-1,3,5-triazine, 2-amino-4-(N-methyl-p-(4'-hydroxyphenylazo)anilino)-6-isopropenyl-1,3,5-triazine, 2-amino-4-(m-(3'-methyl-1'-phenyl-5'-hydroxy-4'-pyrazolylazo)anilino)-6-isopropenyl-1,3,5-triazine, 2-amino-4-(N-methyl-p-(3'-methyl-1'-phenyl-5'-hydroxy-4'-pyrazolylazo)anilino)-6-isopropenyl-1,3,5-triazine, 2-amino-4-(p-phenylazoanilino)-6-isopropenyl-1,3,5-triazine, 4-phenylazo-7-(meth)acryloylamido-1-naphthol; anthraquinone-based polymerizable coloring matters such as 1,5-bis((meth)acryloylamino)-9,10-anthraquinone, 1-(4'-vinylbenzoylamido)-9,10-anthraquinone, 4-amino-1-(4'-vinylbenzoylamido)-9,10-anthraquinone, 5-amino-1-(4'-vinylbenzoylamido)-9,10-anthraquinone, 8-amino-1-(4'-vinylbenzoylamido)-9,10-anthraquinone, 4-nitro-1-(4'-vinylbenzoylamido)-9,10-anthraquinone, 4-hydroxy-1-(4'-vinylbenzoylamido)-9,10-anthraquinone, 1-(3'-vinylbenzoylamido)-9,10-anthraquinone, 1-(2'-vinylbenzoylamido)-9,10-anthraquinone, 1-(4'-isopropenylbenzoylamido)-9,10-anthraquinone, 1-(3'-isopropenylbenzoylamido)-9,10-anthraquinone, 1-(2'-isopropenylbenzoylamido)-9,10-anthraquinone, 1,4-bis(4'-vinylbenzoylamido)-9,10-anthraquinone, 1,4-bis(4'-isopropenylbenzoylamido)-9,10-anthraquinone, 1,5'-bis(4'-vinylbenzoylamido)-9,10-anthraquinone, 1,5-bis(4'-isopropenylbenzoylamido)-9,10-anthraquinone, 1-methylamino-4-(3'-vinylbenzoylamido)-9,10-anthraquinone, 1-methylamino-4-(4'-vinylbenzoyloxy ethylamino)-9,10-anthraquinone, 1-amino-4-(3'-vinylphenylamino)-9,10-anthraquinone-2-sulfonic acid, 1-amino-4-(4'-vinylphenylamino)-9,10-anthraquinone-2-sulfonic acid, 1-amino-4-(2'-vinylbenzylamino)-9,10-anthraquinone-2-sulfonic acid, 1-amino-4-(3'-(meth)acryloylaminophenylamino)-9,10-anthraquinone-2-sulfonic acid, 1-amino-4-(3'-(meth)acryloylaminobenzylamino)-9,10-anthraquinone-2-sulfonic acid, 1-(β-ethoxycarbonylallylamino)-9,10-anthraquinone, 1-(β-carboxyallylamino)-9,10-anthraquinone, 1,5-di-(β-carboxyallylamino)-9,10-anthraquinone, 1-(β-isopropoxy carbonylallylamino)-5-benzoylamido-9,10-anthraquinone, 2-(3'-(meth)acryloylamido-anilino)-4-(3'-(3"-sulfo-4"-aminoanthraquinon-1"-yl)-amino-anilino)-6-chloro-1,3,5-triazine, 2-(3'-(meth)acryloylamido-anilino)-4-(3'-(3"-sulfo-4"-aminoanthraquinon-1"-yl)-amino-anilino)-6-hydrazino-1,3,5-triazine, 2,4-bis-((4"-methoxyanthraquinon-1"-yl)-amino)-6-(3'-vinylanilino)-1,3,5-triazine, and 2-(2'-vinylphenoxy)-4-(4'-(3"-sulfo-4"-aminoanthraquinon-1"-yl-amino)-anilino)-6-chloro-1,3,5-triazine; nitro-based polymerizable coloring matters such as o-nitroanilinomethyl (meth)acrylate; phthalocyanine-based polymerizable coloring matters such as (meth)acryloylated tetraaminocopper phthalocyanine, and (meth)acryloylated (dodecanoylated tetraaminocopper phthalocyanine); and the like. The polymerizable coloring matters may be used alone, or in admixtures of two or more thereof.

(Polymerizable Ultraviolet Ray Absorbing Coloring Matter)

The polymerizable ultraviolet ray absorbing coloring matter is added to the composition for the purpose of imparting ultraviolet ray absorptivity to the ophthalmic lens and the like and coloring the same.

Examples of the polymerizable ultraviolet ray absorbing coloring matter include benzophenone-based polymerizable ultraviolet ray absorbing coloring matters such as 2,4-dihydroxy-3 (p-styrenoazo)benzophenone, 2,4-dihydroxy-5-(p-styrenoazo)benzophenone, 2,4-dihydroxy-3-(p-(meth)acryloyloxymethylphenylazo)benzophenone, 2,4-dihydroxy-5-(p-(meth)acryloyloxymethylphenylazo)benzophenone, 2,4-dihydroxy-3-(p-(meth)acryloyloxyethylphenylazo)benzophenone, 2,4-dihydroxy-5-(p-(meth)acryloyloxyethylphenylazo)benzophenone, 2,4-dihydroxy-3-(p-(meth)acryloyloxypropylphenylazo)benzophenone, 2,4-dihydroxy-5-(p-(meth)acryloyloxypropylphenylazo)benzophenone, 2,4-dihydroxy-3-(o-(meth)acryloyloxymethylphenylazo)benzophenone, 2,4-dihydroxy-5-(o-(meth)acryloyloxymethylphenylazo)benzophenone, 2,4-dihydroxy-3-(o-(meth)acryloyloxyethylphenylazo)benzophenone, 2,4-dihydroxy-5-(o-(meth)acryloyloxyethylphenylazo)benzophenone, 2,4-dihydroxy-3-(o-(meth)acryloyloxypropylphenylazo)benzophenone, 2,4-dihydroxy-5-(o-(meth)acryloyloxypropylphenylazo)benzophenone, 2,4-dihydroxy-3-(p-(N,N-di(meth)acryloyloxyethylamino)phenylazo)benzophenone, 2,4-dihydroxy-5-(p-(N,N-di(meth)acryloyloxyethylamino)phenylazo)benzophenone, 2,4-dihydroxy-3-(o-(N,N-di(meth)acryloyloxyethylamino)phenylazo)benzophenone, 2,4-dihydroxy-5-(o-(N,N-di(meth)acryloylethylamino)phenylazo)benzophenone, 2,4-dihydroxy-3-(p-(N-ethyl-N-(meth)acryloyloxyethylamino)phenylazo)benzophenone, 2,4-dihydroxy-5-(p-(N-ethyl-N-(meth)acryloyloxyethylamino)phenylazo)benzophenone, 2,4-dihydroxy-3-(o-(N-ethyl-N-(meth)acryloyloxyethylamino)phenylazo)benzophenone, 2,4-dihydroxy-5-(o-(N-ethyl-N-(meth)acryloyloxyethylamino)phenylazo)benzophenone, 2,4-dihydroxy-3-(p-(N-ethyl-N-(meth)acryloylamino)phenylazo)benzophenone, 2, 4-dihydroxy-5-(p-(N-ethyl-N-(meth)acryloylamino)phenylazo)benzophenone, 2,4-dihydroxy-3-(o-(N-ethyl-N-(meth) acryloylamino)phenylazo)benzophenone, 2,4-dihydroxy-5-(o-(N-ethyl-N-(meth)acryloylamino)phenylazo)benzophenone, 2,4-dihydroxy-5-(4-(2-(N-(2-methacryloyloxyethyl)carbamoyloxy)ethyl)phenylazo)benzophenone, and 2,4-dihydroxy-5-(4-(2-(N-(2-acryloyloxyethyl)carbamoyloxy)ethyl)phenylazo)benzophenone; and benzoic acid-based polymerizable ultraviolet ray absorbing coloring matters such as phenyl 2-hydroxy-4-(p-styrenoazo)benzoate. The polymerizable ultraviolet ray absorbing coloring matters may be used alone, or in admixtures of two or more thereof.

The total percentage of the polymerizable ultraviolet ray absorbing agent, polymerizable coloring matter and polymerizable ultraviolet ray absorbing coloring matter used in the composition may be preferably no less than 0.01%

by mass and no greater than 3% by mass, and more preferably no less than 0.01% by mass and no greater than 2% by mass. By employing the percentage of the polymerizable ultraviolet ray absorbing agents, polymerizable coloring matter and polymerizable ultraviolet ray absorbing coloring matter used of no less than 0.01% by mass, the ultraviolet ray absorptive and coloring effect may be sufficiently exhibited. On the other hand, by employing the percentage of these components of no greater than 3% by mass, the impairment of the mechanical strength and transparency of the ophthalmic lens and the like may be suppressed, while reducing toxicity to living organisms. Furthermore, if the moisture content of the lens is low, and elution of the components is not observed, then non-polymerizable components such as 2-(4,6-diphenyl-1,3,5-triazin-2-yl)-5-(hexyloxy)phenol, and 2-(5-chloro-2H-benzotriazol-2-yl)-6-(1,1-dimethylethyl)-4-methylphenol may be used.

((E) Crosslinking Agent)

The crosslinking agent may be added as a component (E), in order to modify the crosslinking density, flexibility and rigidity of the resultant ophthalmic lenses and the like. Examples of the crosslinking agent (E) include allyl methacrylate, vinyl methacrylate, 4-vinylbenzyl methacrylate, 3-vinylbenzyl methacrylate, methacryloyloxyethyl acrylate, ethylene glycol dimethacrylate, diethylene glycol dimethacrylate, diethylene glycol diallyl ether, triethylene glycol dimethacrylate, tetraethylene glycol dimethacrylate, propylene glycol dimethacrylate, dipropylene glycol dimethacrylate, butanediol dimethacrylate, trimethylolpropane trimethacrylate, 2,2-bis(p-methacryloyloxyphenyl)hexafluoropropane, 2,2-bis(m-methacryloyloxyphenyl)hexafluoropropane, 2,2-bis(o-methacryloyloxyphenyl)hexafluoropropane, 2,2-bis(p-methacryloyloxyphenyl)propane, 2,2-bis(m-methacryloyloxyphenyl)propane, 2,2-bis(o-methacryloyloxyphenyl)propane, 1,4-bis(2-methacryloyloxyhexafluoroisopropyl)benzene, 1,3-bis(2-methacryloyloxyhexafluoroisopropyl)benzene, 1,2-bis(2-methacryloyloxyhexafluoroisopropyl)benzene, 1,4-bis(2-methacryloyloxyisopropyl)benzene, 1,3-bis(2-methacryloyloxyisopropyl)benzene, 1,2-bis(2-methacryloyloxyisopropyl)benzene and the like. The crosslinking agents may be used alone, or in admixtures of two or more thereof.

The percentage of the crosslinking agent used in the composition may be preferably no less than 0.05% by mass and no greater than 1% by mass, and more preferably no less than 0.1% by mass and no greater than 0.8% by mass. By employing the percentage of the crosslinking agent used of no less than 0.05% by mass, the impairment of the mechanical strength and durability of the ophthalmic lens may be suppressed. On the other hand, by employing the percentage of the crosslinking agent of no greater than 1% by mass, the modification of the flexibility and the like may be ensured.

((F) Polymerization Initiator)

It is preferred that the composition according to the present invention further contains (F) a polymerization initiator for curing of the composition. By blending the polymerization initiator (F) into the composition, the composition can be cured by simple operations such as heating and/or irradiation with an ultraviolet ray or a visible light ray (hereinafter, merely referred to as "light").

Thermal polymerization initiators used for the polymerization by heating include, for example, 2,2'-azobisisobutyronitrile, 2,2'-azobis(2,4-dimethylvaleronitrile), benzoyl peroxide, t-butyl hydroperoxide, cumene hydroperoxide, lauroyl peroxide, t-butyl peroxyhexanoate, 3,5,5-trimethylhexanoyl peroxide and the like. These radical polymerization initiators may be used alone, or in admixtures of two or more thereof. The amount of the radical polymerization initiator used is preferably no less than 0.001 parts by mass and no greater than 2 parts by mass, and more preferably no less than 0.01 parts by mass and no greater than 1 part by mass, with respect to 100 parts by mass of the total polymerization components.

Examples of photopolymerization initiators used for the polymerization by light irradiation include phosphine oxide-based photopolymerization initiators such as 2,4,6-trimethylbenzoyldiphenylphosphine oxide (TPO), and bis(2,4,6-trimethylbenzoyl)-phenylphosphine oxide; benzoin-based photopolymerization initiators such as methyl orthobenzoylbenzoate, methyl benzoylformate, benzoin methyl ether, benzoin ethyl ether, benzoin isopropyl ether, benzoin isobutyl ether, and benzoin n-butyl ether; phenone-based photopolymerization initiators such as 2-hydroxy-2-methyl-1-phenylpropan-1-one (HMPPO), p-isopropyl-$\alpha$-hydroxyisobutyl phenone, p-t-butyltrichloroacetophenone, 2,2-dimethoxy-2-phenylacetophenone, $\alpha$,$\alpha$-dichloro-4-phenoxyacetophenone, and N,N-tetraethyl-4,4-diaminobenzophenone; 1-hydroxycyclohexyl phenyl ketone; 1-phenyl-1,2-propane dione-2-(o-ethoxycarbonyl)oxime; thioxanthone-based photopolymerization initiators such as 2-chlorothioxanthone, and 2-methylthioxanthone; dibenzosuberone; 2-ethylanthraquinone; benzophenone acrylate; benzophenone; benzil, and the like. The photopolymerization initiators may be used alone, or in admixtures of two or more thereof. Further, a photosensitizer may be used in conjunction with the photopolymerization initiator. The percentage of each of the photopolymerization initiator and the photosensitizer used is preferably no less than 0.001 parts by mass and no greater than 2 parts by mass, and more preferably no less than 0.01 parts by mass and no greater than 1 part by mass, with respect to 100 parts by mass of the total polymerization components.

In any case, if the amount of the polymerization initiators is too high, then the resultant cured material may become excessively soft or brittle. Whereas, if the amount of the initiators is too low, then the amount of the monomers and oligomers remaining in the resultant cured material may be increased, and consequently, a surface of the cured material may exhibit stickiness.

Cured Material

The cured material according to the present invention is a cured material which is obtained by curing the composition. Since the cured material contains a polymer having the ionic compound (A) incorporated into its polymer chain as the monomer, the cured material has the superior oxygen permeability and long-term sustainable antibacterial property in combination, as described above.

In methods for curing the composition, each polymerization component in the composition may be copolymerized to permit curing by subjecting the composition to at least one selected from the group consisting of the irradiation with ultraviolet ray and/or visible light ray, and heating. In addition, other methods for curing the composition such as the copolymerization by electron beam irradiation instead of the ultraviolet ray irradiation may be available.

In making the cured material, a bulk polymerization method or solution polymerization method is employed. It should be noted that in the bulk polymerization method, viscosity of the system may steeply increase as the polymerization proceeds; in such a system of high viscosity, the monomer components can hardly diffuse, and therefore the monomers failed to participate in the polymerization reaction often remain unpolymerized. On the other hand, in the solution polymerization method, since solvents are not involved in the reaction, they often remain in the polymer. In the production of contact lens or the like, which is a medical instrument, in order to reduce these residual materials as much as possible, the cured material is subjected to a treatment in which a polymer material obtained by curing are immersed in water or an organic solvent or a mixed solution thereof, and preferably the immersion procedure is repeated, to elute and remove the residual materials from the polymer material. The solvent used in the treatment may include an aqueous solution containing inorganic compounds dissolved, such as physiological saline, or a mixed solution thereof with an organic solvent.

When the cured material according to the present invention is used as a material for ophthalmic lenses, the composition may be cured in a mold casting process. When the composition is heated and polymerized in the mold casting process, an ophthalmic lens material may be produced by charging the polymerizable composition containing the thermal polymerization initiator as described above into a mold corresponding to a shape of the desired material for ophthalmic lens, gradually heating the mold to polymerize the composition, and applying mechanical processing such as cutting processing and polishing processing to the obtained cured material as needed. The cutting may be conducted over the entire surface of one side or both sides of the cured material, or over a part of the surface of one side or both sides of the cured material.

Heating temperature during the heating of the composition in the mold is preferably no less than 50°C and no greater than 150°C, and more preferably no less than 60°C and no greater than 140°C. In addition, heating time during the heating of the composition in the mold is preferably no less than 10 min and no greater than 120 min, and more preferably no less than 20 min and no greater than 60 min. By employing the heating temperature in the mold of no less than 50°C, the polymerization time may reduced, whereas by employing the heating time of no less than 10 min, the reduction of the residual monomer components can be achieved. On the other hand, by employing the heating temperature in the mold of no greater than 150°C or the heating time of no greater than 120 min, suppression of the evaporation of each polymerization component as well as prevention of the deformation of the mold can be achieved.

When the composition is polymerized in the mold casting process by irradiation with light, the ophthalmic lens material may be produced by charging the composition containing the above-described photopolymerization initiator into a transparent mold corresponding to a shape of the desired ophthalmic lens material, subsequently irradiating the mold with the light to polymerize the composition, and applying mechanical processing such as cutting processing and polishing processing to the obtained cured material as needed. In such polymerization with light irradiation, as with the case of the polymerization by heating, the cutting may be conducted over the entire surface of one side or both sides of the cured material, or over a part of the surface of one side or both sides of the cured material.

The material entities of the mold used in the polymerization by light irradiation are not particularly limited as long as they are able to transmit the light necessary for the polymerization and curing, and general-purpose resins such as polypropylene, polystyrene, nylon, polyester are preferred, and glass may be used. By molding and processing these materials, molds with the desired shape can be prepared.

After charging the composition containing the respective polymerization components into such a mold, the polymerization is performed by irradiating the composition with the light. The wavelength range of the irradiating light may be chosen depending on the functions of the ophthalmic lens materials. However, the type of the photopolymerization initiator used must be chosen in accordance with the wavelength range of the irradiating light. The illuminance of the light is preferably no less than 0.1 $mW/cm^2$ and no greater than 100 $mW/cm^2$. Stepwise irradiation with a plurality of

lights of different illuminance may be applied. Moreover, irradiation time of the light is preferably no less than 1 min. By employing such a predetermined illuminance and irradiation time, it is possible to sufficiently cure the polymerizable composition, while preventing the deterioration of the mold material. Furthermore, heating may be applied to the polymerizable composition concurrently with the irradiation with the light, thereby promoting the polymerization reaction, and enabling easy formation of the copolymers.

In order to improve the surface characteristic of the cured material used for the ophthalmic lens material and the like, a low temperature plasma treatment, an atmospheric pressure plasma treatment, a corona discharge treatment, or the like may be applied. By applying the low temperature plasma treatment, ophthalmic lenses with still superior water wettability and/or stain resistance can be obtained. The low temperature plasma treatment may be conducted in an atmosphere of a rarefied gas such as alkanes and fluorine-substituted alkanes both having 1 to 6 carbon atoms, nitrogen, oxygen, carbon dioxide, argon, hydrogen, air, water, silane or any mixture thereof. Particularly, the low temperature plasma treatment is preferably conducted in an atmosphere of a rarefied gas of oxygen alone, carbon dioxide alone, or a mixture of oxygen with water, tetrafluoromethane, organic silanes, methane, nitrogen and the like for the reason that the physical surface modification effect by ion etching and the chemical surface modification effect by radical implantation are expected. The low temperature plasma treatment may be conducted under a reduced pressure or ambient pressure. In the low temperature plasma treatment, a radio-frequency wave RF (for example, 13.56 MHz), audio-frequency wave AF (for example, 15.0 to 40.0 KHz), or microwave (for example, 2.45 GHz) is used, and the surface modification effect can be controlled by appropriately adjusting its output, treatment time, and gas concentration.

Since the cured material contains a polymer having the ionic compound (A) incorporated into its polymer chain as the monomer as described above, the cured material has the superior oxygen permeability and the long-term sustainable antibacterial property in combination. Therefore, the cured material can be used as ophthalmic lens materials, medical equipment materials such as dialysis membranes, catheters, tubes, stents, blood bags, compresses, and plasters, as well as materials for films and the like.

Hydrogel

The hydrogel according to the present invention is a hydrogel containing the cured material described above. More specifically, the hydrogel can be obtained by swelling the cured material as a base material with water. The water components for swelling the cured material include distilled water, and aqueous solutions having inorganic substances dissolved, such as physiological saline, may be used.

Since the hydrogel includes the cured material which contains a polymer having the ionic compound (A) incorporated into its polymer chain as the monomer, the hydrogel has the superior oxygen permeability and the long-term sustainable antibacterial property in combination, as described above.

Therefore, the hydrogel can be preferably used for ophthalmic lens materials as well as medical equipment materials such as dialysis membranes, catheters, tubes, stents, blood bags, compresses, plasters, and additionally, films and the like.

Ophthalmic Lens

The ophthalmic lens according to the present invention is formed of the hydrogel as described above. Therefore, the ophthalmic lens has the superior oxygen permeability and the long-term sustainable antibacterial property in combination. The ophthalmic lens specifically includes contact lenses as well as intraocular lenses, artificial corneas, cornea onlays, cornea inlays and the like.

[Examples]

Hereinafter, the present invention will be explained in more detail by way of Examples, but the present invention is by no means limited by Examples.

In the following, meanings of the abbreviations for the compounds used in Examples are presented.

(A) Ionic Compounds

TrisAA-I: a compound represented by the above formula (1) in which P-$Q^+$- is represented by the formula (1-1); Z is represented by the formula (3); $X^{m-}$ represents $I^-$; and n is 1 (additionally, in the formula (1-1), $R^1$ represents a hydrogen atom; Y represents -O-; and n1 is 2. Further, in the formula (3), n3 is 3.)

TrisAA-TFSI: a compound represented by the above formula (1) in which P-$Q^+$- is represented by the formula (1-1); Z is represented by the formula (3); $X^{m-}$ represents TFSI (the anion represented by the above formula (5)); and n is 1 (additionally, in the formula (1-1), $R^1$ represents a hydrogen atom; Y represents -O-; and n1 is 2. Further, in the formula

(3), n3 is 3. In the formula (5), n4 is 0.)

TrisVI-I: a compound represented by the above formula (1) in which P-$Q^+$- is represented by the formula (1-2); Z is represented by the formula (3); $X^{m-}$ represents $I^-$; and n is 1 (additionally, in the formula (1-2), $R^2$ represents $CH_2$=CH-. Further, in the formula (3), n3 is 3.)

TrisVI-TFSI: a compound represented by the above formula (1) in which P-$Q^+$- is represented by the formula (1-2), Z is represented by the formula (3), $X^{m-}$ represents TFSI (the anion represented by the above formula (5)), and n is 1 (additionally, in the formula (1-2), $R^2$ represents $CH_2$=CH-. Further, in the formula (3), n3 is 3. In the formula (5), n4 is 0.)

(B) Hydrophilic Monomers

HEMA: 2-hydroxyethyl methacrylate
DMAA: N,N-dimethylacrylamide
N-MMP: N-methyl-3-methylene-2-pyrrolidinone (1-methyl-3-methylene-2-pyrrolidone)

(C) Silicone Compounds

Urethane-containing siloxane macromer: a compound represented by the above formula (C-1)
TRIS: tris(trimethylsiloxy)silylpropyl methacrylate

(D) Crosslinking Agent

EDMA: ethylene glycol dimethacrylate

(E) Polymerization Initiator

HMPPO: 2-hydroxy-2-methyl-1-phenylpropan-1-one
Ionic Compounds Used in Comparative Examples

ADDA-Br: 2-acryloyloxyethyldimethyldodecylammonium bromide) (a compound represented by the following formula (12))

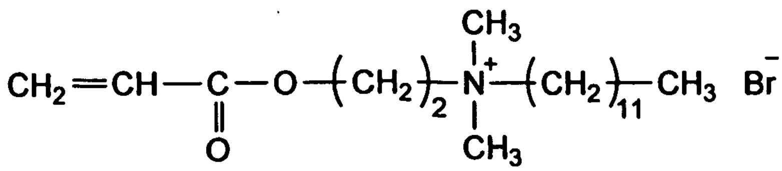

( 12 )

BVI-Br: 3-butyl-1-vinylimidazolium bromide (a compound represented by the following formula (13))

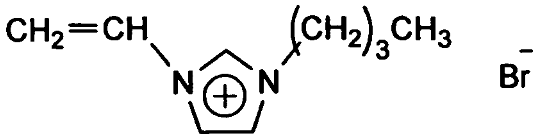

( 13 )

ADCA-TFSI: 2-acryloyloxyethyldimethylcetylammonium bis(trifluoromethanesulfonyl)imide (a compound represented by the following formula (14))

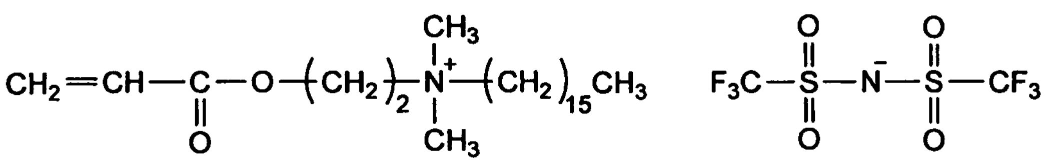

( 14 )

Example 1 (Synthesis of TrisAA-I)

3-Iodopropyltris(trimethylsiloxy)silane (25.63 g), dimethylaminoethyl acrylate (7.89 g), toluene (20 g) were mixed and dissolved, and the mixture was stirred at 80°C for about 17 hrs, to allow the reaction to proceed. To the reaction mixture was added 400 ml of diethyl ether to yield a clear solution, and the clear solution was allowed to stand at room temperature for some time. Deposited white precipitates were filtered off with a Kiriyama funnel. To the white solid on the filter paper was added 100 ml of diethyl ether to wash the white solid, and diethyl ether was removed by filtration. This procedure was repeated three times. The white solid was dried under vacuum to yield 24.68 g of TrisAA-1.

Example 2 (Synthesis of TrisAA-TFSI)

TrisAA-I (20.06 g) was dissolved in dichloromethane (50 ml), and ion exchanged water (50 ml) was further added. Lithium bis(trifluoromethanesulfonyl)imide (14.39 g) was added to the mixture, and the mixture was intensively stirred at room temperature for about 5 hrs. The reaction mixture was transferred to a separatory funnel, and to this was added 200 ml of dichloromethane and 300 ml of ion exchanged water were added and the mixture was shaken intensively and phases were separated. The dichloromethane layer was recovered, and was shaken intensively with 300 ml of ion exchanged water and phases were separated, thereby washing the dichloromethane layer. The washing was further repeated five times. The dichloromethane layer was recovered, an appropriate amount of anhydrous sodium sulfate was added, and the mixture was stirred and allowed to stand for some time. Sodium sulfate was removed by filtration and the filtrate was recovered, and then dichloromethane was distillated away under reduced pressure to yield 23.97 g of TrisAA-TFSI as a white solid.

Example 3 (Synthesis of TrisVI-I)

3-Iodopropyltris(trimethylsiloxy)silane (9.84 g) and 1-vinylimidazole (2.40 g) were mixed to form a suspension, and the suspension was stirred at 80°C for about 17 hrs, to allow the reaction to proceed. To the reaction mixture was added 30 ml of acetonitrile to yield a clear solution, and the clear solution was transferred to a separatory funnel, and to this were added 100 ml of acetonitrile and 120 ml of n-hexane and the mixture was shaken intensively and phases were separated. The acetonitrile layer was recovered, and was shaken intensively with 120 ml of n-hexane and phases were separated, thereby washing the acetonitrile layer. The washing was further repeated twice. The acetonitrile layer was recovered, and acetonitrile was distilled off by applying a vacuum. To the residue was added about 80 ml of ion exchanged water, and the mixture was intensively stirred, and the deposited precipitates were filtered off with a Kiriyama funnel. To the white solid on top of the filter paper was added 80 ml of ion exchanged water to wash the white solid, and the ion exchanged water was removed by filtration. The white solid was dried under vacuum to yield 11.80 g of TrisVI-I.

Example 4 (Synthesis of TrisVI-TFSI)

TrisVI-I (9.31 g) was dissolved in 15 ml of dichloromethane, and 20 ml of ion exchanged water was further added to the solution. To this was added lithium bis(trifluoromethanesulfonyl)imide (7.39 g), and the mixture was intensively stirred at room temperature for about 18 hrs. The reaction mixture was transferred to a separatory funnel, and to this were added 100 ml of dichloromethane and 100 ml of ion exchanged water and the mixture was shaken intensively and phases were separated. The dichloromethane layer was recovered, and was shaken intensively with 150 ml of ion exchanged water and phases were separated, thereby washing the dichloromethane layer. The washing was further repeated five times. The dichloromethane layer was recovered, an appropriate amount of anhydrous sodium sulfate was added, and the mixture was stirred and allowed to stand for some time. Sodium sulfate was removed by filtration, and the filtrate was recovered, and dichloromethane was distillated away under reduced pressure to yield 10.19 g of TrisAA-TFSI as a white solid.

Example 5

DMAA (10 parts by mass) and N-MMP (35 parts by mass) as the hydrophilic monomer (B), the urethane-containing siloxane macromer (33 parts by mass) and TRIS (22 parts by mass) as the silicone compound (C), EDMA (0.4 parts by mass) as the crosslinking agent (E), and HMPPO (0.4 parts by mass) as the polymerization initiator (F) were homogeneously mixed and dissolved, to prepare a polymerizable composition A. The polymerizable composition A (95 parts by mass) and TrisAA-TFSI (5 parts by mass) were mixed and dissolved to prepare a blend liquid. The blend liquid was injected into a mold for contact lenses made of a polypropylene resin, and polymerized for about 20 min at the illuminance of about 10 mW/$cm^2$ by using an ultraviolet ray irradiation apparatus equipped with an ultraviolet ray lamp (ultra-violet curing unit UBX0302-03 from Eye Graphics Co., Ltd.), which had the main wavelength at 365 nm. After

releasing from the mold, the lens was subjected to a plasma treatment (apparatus; low temperature asher PA-102AT from Kyoto Denshi Keisoku Co., Ltd.; condition; 0.8 Torr $O_2$, output: 50 W, treatment time: 2 min), and subsequently immersed in distilled water (2 ml per lens). After about 16 hrs, the lens was transferred to the same amount ratio (i.e., 2 ml per lens) of physiological saline. After another about 6 hrs, the lens was transferred to the same amount ratio of fresh physiological saline, and was autoclaved to yield a transparent soft contact lens (hydrogel).

Example 6

A solution obtained by mixing and dissolving the polymerizable composition A (100 parts by mass) and TrisAA-TFSI (0.2 parts by mass) was employed as the blend liquid, and a soft contact lens was prepared in a similar manner to Example 5.

Example 7

A solution obtained by mixing and dissolving the polymerizable composition A (90 parts by mass) and TrisAA-TFSI (1 part by mass) was employed as the blend liquid, and a soft contact lens was prepared in a similar manner to Example 5.

Example 8

A solution obtained by mixing and dissolving the polymerizable composition A (80 parts by mass) and TrisAA-TFSI (20 parts by mass) was employed as the blend liquid, and a soft contact lens was prepared in a similar manner to Example 5.

Example 9

A solution obtained by mixing and dissolving the polymerizable composition A (95 parts by mass) and TrisVI-TFSI (5 parts by mass) was employed as the blend liquid, and a soft contact lens was prepared in a similar manner to Example 5.

Example 10

A solution obtained by mixing and dissolving the polymerizable composition A (95 parts by mass) and TrisAA-I (5 parts by mass) was employed as the blend liquid, and a soft contact lens was prepared in a similar manner to Example 5.

Example 11

A solution obtained by mixing and dissolving the polymerizable composition A (95 parts by mass) and TrisVI-I (5 parts by mass) was employed as the blend liquid, and a soft contact lens was prepared in a similar manner to Example 5.

Example 12

DMAA (35 parts by mass) as the hydrophilic monomer (B), EDMA (0.3 parts by mass) as the crosslinking agent (E), and HMPPO (0.25 parts by mass) as the polymerization initiator (F) were homogeneously mixed and dissolved, to prepare a polymerizable composition B. The polymerizable composition B (35.55 parts by mass) and TrisAA-TFSI (65 parts by mass) were mixed and dissolved, to prepare a blend liquid. The blend liquid was injected into a plate mold made of a polypropylene resin, and polymerized for about 20 min at the illuminance of about 10 $mW/cm^2$ by using an ultraviolet ray irradiation apparatus equipped with an ultraviolet ray lamp (ultra-violet curing unit UBX0302-03 from Eye Graphics Co., Ltd.), which had the main wavelength at 365 nm. After releasing from the mold, a plate-shaped cured material with a diameter of about 20 mm and a thickness of about 0.2 mm was obtained. The cured material was immersed in distilled water (10 ml per plate). After about 16 hrs, the obtained hydrogel with a diameter of about 13 mm was cut out, and transferred to the same amount ratio of fresh physiological saline. After another about 8 hrs, the hydrogel was transferred to the same amount ratio of fresh physiological saline, and was autoclaved.

Comparative Example 1

A soft contact lens was prepared in a similar manner to Example 1 by using the polymerizable composition A alone as the blend liquid.

Comparative Example 2

In order to obtain a blend liquid, 95 parts by mass of the polymerizable composition A and 5 parts by mass of BVI-Br were mixed. However, homogeneous dissolution was not achieved but a cloud blend liquid was obtained, and accordingly preparation of transparent lens failed.

Comparative Example 3

In order to obtain a blend liquid, 95 parts by mass of the polymerizable composition A and 5 parts by mass of ADDA-Br. However, homogeneous dissolution was not achieved but a cloud blend liquid was obtained, and accordingly preparation of transparent lens failed.

Comparative Example 4

A solution obtained by mixing and dissolving 35.55 parts by mass of the polymerizable composition B and 65 parts by mass of ADCA-TFSI was employed as the blend liquid, and a plate-type hydrogel was prepared in a similar manner to Example 7.

Evaluation

<Comparison of Miscibility>

In Examples 5 to 12, transparent lenses could be prepared, whereas in Comparative Examples 2 and 3, preparation of transparent lens failed. It has been indicated that the incorporation of the silicone site into the molecules improves their miscibility.

<Evaluation of Antibacterial Property>

- Preparation of SCDA Medium

To purified water (400 ml) was added a soy-bean-casein digest agar medium (Nihon Pharmaceutical Co., Ltd.) (16.0 g), and the mixture was subjected to steam sterilization under pressure at 121°C for 20 min.

- Preparation of 500X Dilution Nutrient Broth Medium (1/500 NB Medium)

To purified water (100 ml) was added a nutrient broth medium (Eiken Chemical Co., Ltd.) (1.8 g), and dissolved therein with heating, and then 1 ml of the solution was taken and diluted 500X, and the diluted solution was subjected to steam sterilization under pressure at 121°C for 20 min.

- Preparation of Inocula

(1) A colony was collected from a master plate of a strain *(Staphylococcus aureus* NBRC13276), and then plated on the SCDA medium.
(2) Preculture was conducted at 35 ± 2°C.
(3) The precultured bacteria were suspended in the 1/500 NB medium, and prepared so as to achieve a viable bacterial count of about $10^8$ cfu/ml based on the transmittance at 660 nm. Furthermore, the bacterial culture was diluted with the 1/500 NB medium so as to attain a viable bacterial count of 1.0 x $10^4$ to 1.4 x $10^4$ cfu/ml, and the diluted bacterial culture was used as an inoculum.

The lenses prepared in Examples 5 to 11 and Comparative Example 1 were subjected to the following operation:

(1) A predetermined number of lenses and the inoculum were charged into a vial such that (surface area of the lens)/(inoculum) = 32 $cm^2$/10 ml.
(2) The vial was closed with a cap, and the mixture was subjected to shaking culture at 150rpm for about 24 hrs at 35 ± 2°C.
(3) After completion of the culture, 1 ml of the bacterial culture was took up, and serially diluted with the 1/500 NB

medium, and the diluted bacterial culture was inoculated in the SCDA medium, and cultured at 35 ± 2°C.
(4) Detected colonies were counted, and the viable bacterial count was calculated. The viable bacterial count is shown in Table 1 below for showing the results of the evaluation of the antibacterial property.

[Table 1]

| | Example 5 | Example 6 | Example 7 | Example 8 | Example 9 | Example 10 | Example 11 | Comparative Example 1 |
|---|---|---|---|---|---|---|---|---|
| Survived viable bacterial count (cfu/m 1) | $8.1 \times 10^1$ | $5.7 \times 10^4$ | $1.6 \times 10^3$ | 0 | $2.1 \times 10^1$ | 0 | 0 | $1.1 \times 10^5$ |
| Percentage of survived viable cell with respect to Comparative Example 1(%) | 0.07 | 52 | 1.5 | 0 | 0.02 | 0 | 0 | - |

As shown in the above Table 1, the contact lenses according to Examples 5 to 11 exhibited lower viable bacterial count compared to Comparative Example 1, indicating that the proliferation of the bacteria was suppressed.

<Comparison of Oxygen Permeability>

The plate-type hydrogels according to Example 12 and Comparative Example 4 were used as a measurement sample, their thicknesses were measured, and then each sample was applied to an Industrial Products Research Institute type film oxygen permeability meter from Rika Seiki Kogyo Co., Ltd., and an electric current value, which is a parameter for an amount of permeated oxygen, was measured at 35°C in accordance with the electrode method. A plate of Menicon EX with a thickness of 0.2 mm, which is specified in the ISO standard, was employed as a reference sample, and an electric current value for this plate was measured in a similar manner. By using the oxygen permeability coefficient of the reference sample of $64 \times 10^{-11}$ ($cm^2$/sec) · (ml $O_2$/ml·mmHg) and the following equation, the oxygen permeability coefficient of the measurement samples was calculated.

$$\text{oxygen permeability coefficient } (cm^2/sec)\cdot(ml\ O_2/ml\cdot mmHg) = W \times (IS/IW) \times (TS/TW) \times (PW/PS)$$

W: oxygen permeability coefficient of the reference sample (($cm^2$/sec) · (ml $O_2$/ml · mmHg))
IS: electric current value for the measurement sample (μA)
IW: electric current value for the reference sample (μA)
TS: thickness of the measurement sample (mm)
TW: thickness of the reference sample (mm)
PS: ambient pressure at the time of the measurement of the measurement sample (mmHg)
PW: ambient pressure at the time of the measurement of the reference sample (mmHg)

The oxygen permeability coefficient for the plate-type hydrogel according to Example 12 was $52.1 \times 10^{-11}$ ($cm^2$/sec) · (ml $O_2$/ml · mmHg), whereas the oxygen permeability coefficient for the plate-type hydrogel according to Comparative Example 4 was $28.6 \times 10^{-11}$ ($cm^2$/sec) · (ml $O_2$/ml · mmHg). This indicates that the hydrogel according to the present invention has superior oxygen permeability.

[INDUSTRIAL APPLICABILITY]

As explained above, the ionic compound according to the present invention can provide cured materials maintaining the superior antibacterial property and possessing the superior oxygen permeability. Therefore, the cured material obtained from the composition containing the ionic compound can be widely used, as a material having antibacterial property and oxygen permeability, in ophthalmic lenses, materials for medical instruments and the like.

## Claims

1. An ionic compound represented by the following formula (1):

$$\left[\left(P-Q^{+}\right)_{n} Z\right]\ \frac{n}{m} X^{m-} \qquad (1)$$

wherein, P represents a polymerizable functional group; $Q^+$ represents a cationic group; Z represents a siloxanyl group in which the total atomic weight of a group of constituent atoms is no less than 70 and no greater than 1,000; $X^{m-}$ represents an m-valent anion; and n is an integer of 1 to 10, wherein provided that n is no less than 2, a plurality of Ps and $Q^+$s are each independently as defined above.

2. The ionic compound according to claim 1, wherein n is 1, and $P-Q^+-$ is represented by the following formula (1-1):

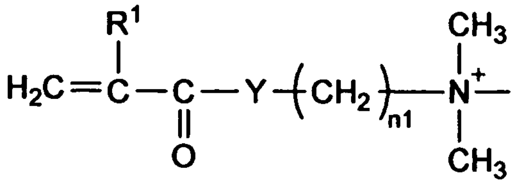

(1-1)

wherein, $R^1$ represents a hydrogen atom or a methyl group; Y represents -O- or -NH-; and n1 is an integer of 1 to 18, the following formula (1-2):

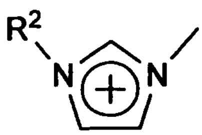

(1-2)

wherein, $R^2$ represents $CH_2=CH-$, $CH_2=CH-CH_2-$, or a group represented by the following formula (2):

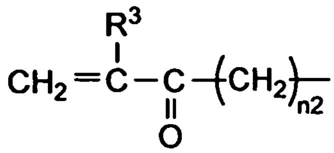

(2)

wherein, $R^3$ represents a hydrogen atom or a methyl group; and n2 is an integer of 0 to 18, or the following formula (1-3):

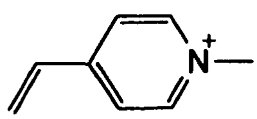

(1-3)

3. The ionic compound according to claim 2, wherein Z represents a group represented by the following formula (3):

$$-\left(CH_2\right)_{n3}-Si\left[OSi\left(CH_3\right)_3\right]_3 \qquad (3)$$

wherein, n3 is an integer of 1 to 10.

4. The ionic compound according to any one of claims 1 to 3, wherein $X^{m-}$ comprises a fluorine atom.

5. The ionic compound according to claim 4, wherein $X^{m-}$ represents at least one selected from the group consisting of the anions represented by the following formula (4) and formula (5):

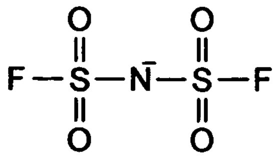

(4)

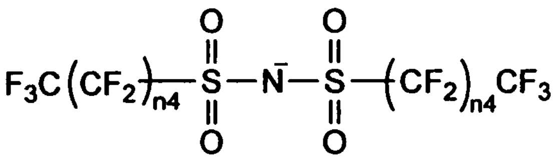

(5)

wherein, n4 is an integer of 0 to 3.

6. A composition comprising the ionic compound (A) according to any one of claims 1 to 5.

7. The composition according to claim 6, further comprising:

(B) a hydrophilic monomer; and
(C) a silicone compound.

8. A cured material obtained from the composition according to claim 6 or 7.

9. A hydrogel comprising the cured material according to claim 8.

10. An ophthalmic lens formed of the hydrogel according to claim 9.

## INTERNATIONAL SEARCH REPORT

International application No.
PCT/JP2010/060061

A. CLASSIFICATION OF SUBJECT MATTER
*C08F20/26*(2006.01)i, *C07C311/48*(2006.01)i, *C07F7/10*(2006.01)i, *G02C7/04*(2006.01)i

According to International Patent Classification (IPC) or to both national classification and IPC

B. FIELDS SEARCHED

Minimum documentation searched (classification system followed by classification symbols)
C08F12/00-38/04, C07C311/48, C07F7/10, G02C7/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| E,A | WO 2010/095478 A1 (Seed Co., Ltd.), 26 August 2010 (26.08.2010), claims 1 to 4 (Family: none) | 1-10 |
| A | JP 1-501474 A (Barnes-Hind, Inc.), 25 May 1989 (25.05.1989), claims 1 to 19 & US 4861840 A & WO 1988/004299 A1 | 1-10 |
| A | JP 2005-508411 A (Bausch & Lomb Inc.), 31 March 2005 (31.03.2005), claims 1 to 28 & US 2003/0100697 A1 & US 2005/0127557 A1 & US 2005/0131189 A1 & US 2005/0131191 A1 & WO 2003/040158 A1 | 1-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 September, 2010 (07.09.10) | 21 September, 2010 (21.09.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/060061

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-282877 A (Fuji Photo Film Co., Ltd.), 19 October 2006 (19.10.2006), claims 1 to 10 (Family: none) | 1-10 |
| A | JP 54-92918 A (Nippon Paint Co., Ltd.), 23 July 1979 (23.07.1979), claims 1, 2 & US 4238609 A & GB 2009748 A & WO 1979/000338 A1 & DE 2852431 A1 | 1-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H476518 B
- JP H6337378 B